# EUROPEAN PATENT APPLICATION

(11) **EP 2 954 896 A1**
(43) Date of publication of application: **16.12.2015**
(21) Application number: 14172399.9
(22) Date of filing: 13.06.2014
(51) Int. Cl.: A61K 31/136, A61K 31/167, A61P 25/00

(54) **Small molecule inhibitors of the E3 Ligase Skp2 in depression and other diseases**

(71) Applicant: Max-Planck-Gesellschaft zur Förderung der Wissenschaften e.V., 80539 München (DE)
(72) Inventor: Gassen, NILS, 80799 Munich (DE); Rein THEO, 80995 Munich (DE)
(74) Representative: Arth, Hans-Lothar

(57) **Abstract**

The present invention relates to Skp2 inhibitors for the use in the therapeutic activation of autophagy and especially for use as an antidepressant. Furthermore the present invention refers to a method of determining if a subject suffering from depression responds to an antidepressant and to a method for screening for novel compounds for the therapeutic activation of autophagy which is suitable for different diseases, such as depression.

## Description

The present invention relates to Skp2 inhibitors for the use in the therapeutic activation of autophagy and especially for use as an antidepressant. Furthermore the present invention refers to a method of determining if a subject suffering from depression responds to an antidepressant t and to a method for screening for novel compounds for the therapeutic activation of autophagy which is suitable for different diseases, such as depression.

### Background of the invention

Depressive disorders comprise one of the most frequent clinical conditions worldwide. Life-time prevalence rates, even when conservatively estimated, range between 10 and 15 percent. These disorders are associated with considerable functional impairment as reflected by increased mortality, even if the high rate of suicides among affected patients is not taken into consideration. The World Health Organization (WHO) concluded from studies conducted by the Harvard Medical School and the World Bank that in the year 2020 depression will be the second leading cause for illness-related disability trailing only cardiovascular disease. Depression, however, is also a major independent risk factor for cardiovascular disease, hypertension, diabetes and osteoporosis. Thus, depression is also a major socio-economic burden.

Autophagosome formation is the key process in autophagy and is highly dynamic in cells. Macroautophagy (autophagy hereafter) is an evolutionally conserved catabolic mechanism in eukaryotes, where excess or damaged organelles and both long-lived and aggregated proteins are cleared. In autophagy, double-membrane vesicle structures, termed autophagosomes, sequester and engulf cytoplasmic components constitutively, or upon nutrient deprivation or stress. The subsequent autophagosome maturation is achieved by fusing with endosomes and/or lysosomes to form autolysosomes, leading to the exposure of the cargo to lysosomal hydrolases for digestion. Autophagy plays a critical role in normal physiology. Autophagy occurs at basal levels in all cells to carry out homeostatic functions and is elevated under starvation or stress conditions. It is pivotal for cell survival during nutrient deprivation, as it functions to recycle intracellular material for new nutrients and energy production. In addition to endogenous substrates, autophagy degrades intracellular pathogens in a selective form of autophagy, termed xenophagy. In this line, autophagy plays an adaptive role in protecting against various pathologies and has been discussed as a potential therapeutic target for diverse diseases (Rubinsztein et al., nature Reviews Drug Discovery 2012; volume 11; 709). Autophagosome induction to enhance autophagy is implicated in the treatment of diseases such as neurodegeneration, cancer, muscular disorders and several infection diseases.

Yeast genetic studies revealed more than 30 autophagy-related (*ATG*) genes, among which 18 Atg proteins (Atg1-10, 12-14, 16-18, 29 and 31) are essential for the autophagosome formation. Most of these *ATG* genes are conserved throughout eukaryotes, termed as "core" autophagy machinery. The core Atg proteins are categorized into four major functional groups: (i) the Atg1/unc-51-like kinase 1 (ULK1) complex including Atg1/ULK1, Atg13, Atg17/FIP200, Atg29, and Atg31; (ii) the class III phosphatidylinositol 3-kinase (PI3K) complex consisting of vacuolar protein sorting (Vps) 34/PI3K, Atg6/Beclin1, Atg14, and Vps15/p150; (iii) transmembrane protein Atg9 and associated Agt2-Atg18/WIPI-1 complex; (iv) two ubiquitin-like protein (Ubl) conjugation systems, which include Ubl proteins Atg8/LC3 and Atg12, an E1-like enzyme Atg7, two E2-like enzyme Atg3 and Atg10, an Atg8 protease (Atg4), the target of Atg12 conjugation (Atg5) and the binding partner Atg16. In yeast the majority of core Atg proteins are recruited to the single phagophore-assembly site (PAS, also termed pre-autophagosomal structure). In mammalian cells, autophagosomes instead initiate from multiple phagophores or isolation membranes (IMs) throughout the cytosol. IMs expand, enfold cytosol and finally close, forming double-membrane vesicles (autophagosomes). The origin of the IM and the complete mechanisms underlying the action of these interacted groups on the formation of autophagosomes remain to be elucidated in detail. Nevertheless, it is known that Beclin-1 is required for the initiation of the formation of the autophagosome in mammalian cells.

FKBPs, or FK506 binding proteins, belong to a protein family with prolyl isomerase activity. This enzymatic activity is shared by the cyclophilins. The amino acid sequence, however, is not related. FKBPs have been identified in many eukaryotes from yeast to humans and function both as chaperones and cochaperones. Cyclophilins and FKBPs together build the immunophilin family.

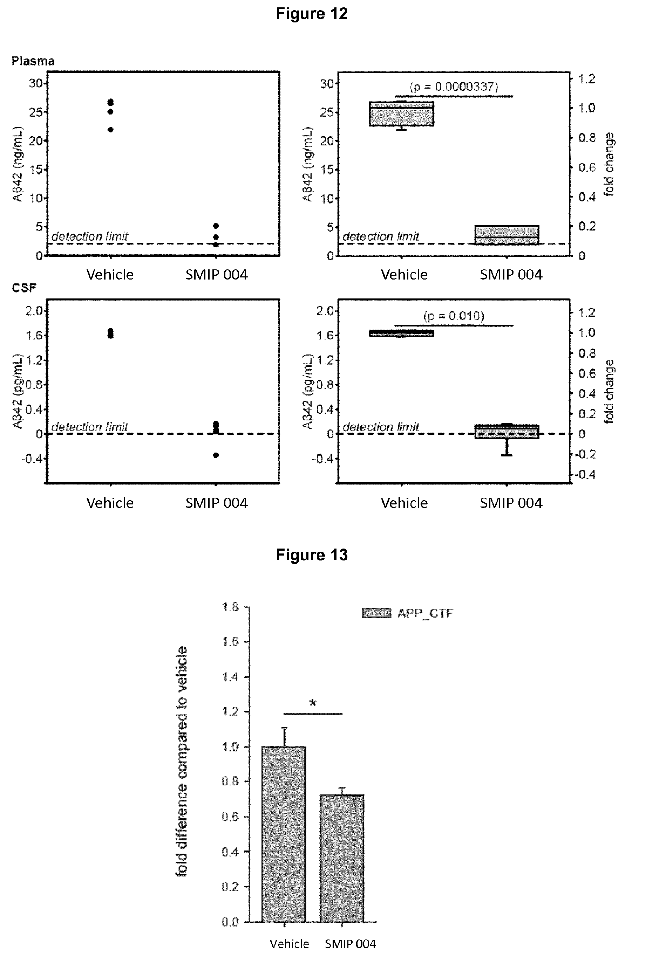

FK506 binding protein 51 (FKBP51) is a regulator of the glucocorticoid receptor, and consequently of the stress hormone axis and stress physiology. Human genetic studies suggested a link of FKBP51 to the antidepressant (AD) response rate. Despite the intimate connection of stress physiology to pathophysiology and treatment of depression the mechanistic role of FKBP51 for AD response has not been elucidated. The inventors could identify FKBP51-directed signalling pathways and downstream autophagic events and show that these pathways are targeted by antidepressants in cells and brain tissue of mice. Based on mechanistic pathway analyses of the depression risk factor FKBP51 the inventors put forward that pharmacological inhibition of the E3 ligase Skp2 could elicit antidepressant-like effects. Skp2 is an ubiquitin ligase that determines the protein stability of the autophagy initiator and executor Beclin1. The inventors could show that FKBP51 recruits the phosphatase PHLPP along with Akt1 to Skp2 and Beclin1. This results in inactivation of Skp2 and therefore stabilization of Beclin1. Results of the present invention also proved evidence in cells, animals and humans that this mechanism could explain why antidepressants need FKBP51 for their pharmacological action. These findings suggest that direct inhibition of Skp2 might also elicit antidepressant-like effects. In fact, experimental data of the present invention show that pharmacological inhibition of Skp2 elicits antidepressant-like effects in mice, as determined by the tail suspension test in two different mouse strains. In addition, inhibition of Skp2 showed the comparable effects on synaptic neurotransmission in brain slices as the antidepressant paroxetine. Furthermore the inventors could show that Skp2 inhibition reduces the levels of soluble Aβ in plasma and cerebrospinal fluid (Fig. 12) and reduces the levels of APP-CTF in the brain (Fig. 13).

The application WO 2009/025854 A1 discloses Skp2 inhibitors interfering with the depletion of the tumor suppressor p27. The compound SMIP004 (N-(4-butyl-2-methyl-phenyl) acetamide) and analogues thereof were identified as inducer of cancer-cell selective apoptosis of human prostate cancer cells. SMIP004 decreased the levels of positive cell cycle regulators, upregulated cyclin-dependent kinase inhibitors, and resulted in G1 arrest, inhibition of colony formation in soft agar, and cell death. However, the mechanism of SMIP004-induced cancer cell selective apoptosis remained unknown. Current results suggest that SMIP004 acts as antiproliferative agent by compromising the ability of cancer cells to withstand environmental stress by interfering with the functioning of mitochondria. There was no hint that Skp2 inhibitors could be useful for increasing stability of the autophagy initiator and executor Beclin1 and therefore for the treatment of a psychiatric disorder, infectious disease, a myopathy or a neurodegenerative disease. Other Skp2 inhibitors, for example SKP2 E3 Ligase Inhibitor I, C1 purchased by Calbiochem, were described by Wu and Co-Authors (Wu et al. Chemistry and Biology; 2012 volume 19, 12; 1515-1524).

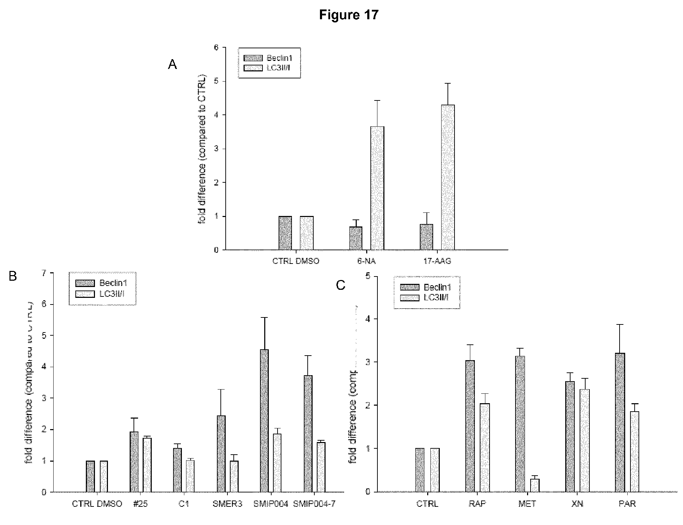

Skp2 inhibitors vary in their cell toxicity (Fig. 17) and not all Skp2 inhibitors induce autophagy. This could be the result of a specific interference with the ubiquitination of different targets of the E3 ligase Skp2. It seems that only some Skp2 inhibitors, such as SMIP004 interfere with the action of Skp2 on Beclin1.

It is the objective of the present invention to provide compounds and/or pharmaceutically acceptable salts thereof which can be used as pharmaceutically active agents for the treatment of a psychiatric disorder, especially of depression. Another objective of the present invention is to provide novel uses of the known pharmaceutically active agent N-(4-butyl-2-methyl-phenyl) acetamide and its analogues.

The objective of the present invention is solved by the teaching of the independent claims. Further advantageous features, aspects and details of the invention are evident from the dependent claims, the description, the figures, and the examples of the present application.

### Description of the invention

Therefore the present invention refers to a compound of the general formula I: 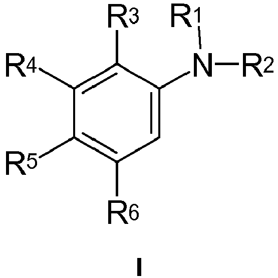 wherein
R¹ and R² are independently of each other selected from -R⁷, -R⁸, -COR⁷, -COR⁸, -CO₂R⁷, -CO₂R⁸, -(CR¹⁰R¹¹)ₙ-R¹², -(CR¹³R¹⁴)ₘ-R¹⁵, -(CR¹⁰R¹¹)ₙ-O-R⁷, -(CR¹³R¹⁴)ₘ-OR ⁸, -(CR¹⁰R¹¹)ₙ-SR⁷, -(CR¹³R¹⁴)ₙ-SR⁸, -(CR¹⁰R¹¹)ₙ-NR⁹R⁷, -(CR¹³R¹⁴)-NR¹⁶R⁸, -CR¹⁰R¹¹-O-CR¹²R¹³R¹⁵, -CR¹⁰R¹¹-S-CR¹²R¹³R¹⁵, -CR¹⁰R¹¹-NR⁹-CR¹²R¹³R¹⁵, -CR¹⁰R¹¹-CR¹²R¹³-R⁷; and R¹ and R² can form together with the nitrogen atom they are connected to a heterocycle selected from: 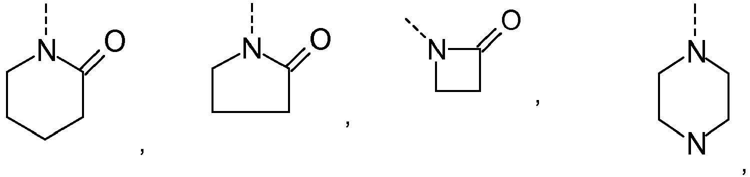 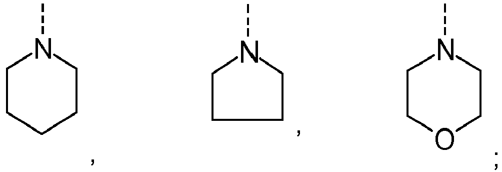 R³ is selected from: -H, -F, -Cl, -Br, -I, -CH₃, -C₂H₅, -C₃H₇, -C₄H₉, -C₅H₁₁, -C₆H₁₃;
R⁵ is selected from: -CH₃, -C₂H₅, -C₃H₇, -C₄H₉, -C₅H₁₁, -C₆H₁₃, -C₇H₁₅, -C₈H₁₇, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -C≡CH, -C≡C-CH₃, -CH₂-C≡CH, -C₂H₄-C≡CH, -CH₂-C≡C-CH₃, -C≡C-C₂H₅, -C≡C-C₃H₇, -CH₂-C≡C-C₂H₅, -C₂H₄-C≡C-CH₃, -C₃H₇-C≡CH, 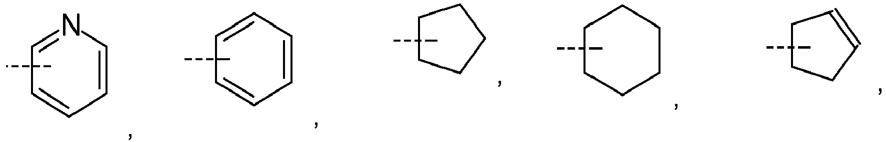 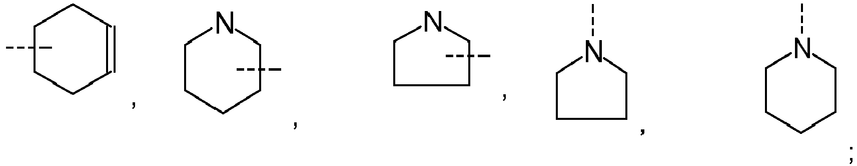 R⁴ represents H or R⁴ and R⁵ form together with the two aromatic carbon atoms they are connected to a cycle selected from: 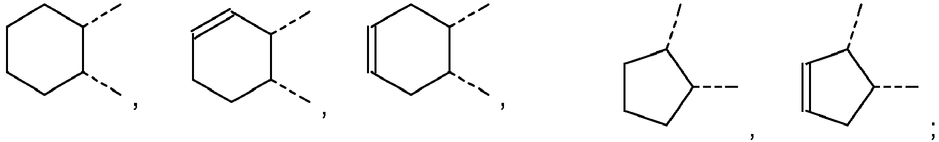 R⁶ represents H or R⁶ and R⁵ form together with the two aromatic carbon atoms they are connected to a cycle selected from: 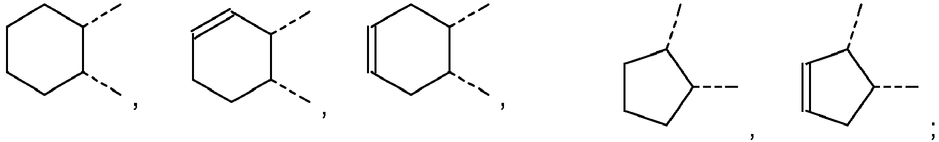 R⁷ and R⁸ are independently of each other selected from: -H, -CH₃, -C₂H₅, -C₃H₇, -C₄H₉, -C₅H₁₁, -C₆H₁₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -CH₂-C≡CH, -C₂H₄-C≡CH, -CH₂-C≡C-CH₃, -CH₂-C≡C-C₂H₅, -C₂H₄-C≡C-CH₃, -C₃H₇-C≡CH;
R¹⁰, R¹¹, R¹², R¹³, R¹⁴ and R¹⁵ are independently of each other selected from: -H, -CH₃, -C₂H₅, -C₃H₇, -C₄H₉, -C₅H₁₁, -C₆H₁₃, -OCH₃, -OC₂H₅, -OC₃H₇, -OC₄H₉, -SCH₃, -SC₂H₅, -SC₃H₇, -SC₄H₉, -NR¹⁸R¹⁹, -NR²⁰R²¹, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -C=CH, -C≡C-CH₃, -CH₂-C≡CH, -C₂H₄-C≡CH, -CH₂-C≡C-CH₃, -C≡C-C₂H₅, -C≡C-C₃H₇, -CH₂-C≡C-C₂H₅, -C₂H₄-C≡C-CH₃, -C₃H₇-C≡CH,
R⁹, R¹⁶, R¹⁸, R¹⁹, R²⁰ and R²¹ are independently of each other selected from -H, -CH₃, -C₂H₅, -C₃H₇, -C₄H₉, -C₅H₁₁, -C₆H₁₃, 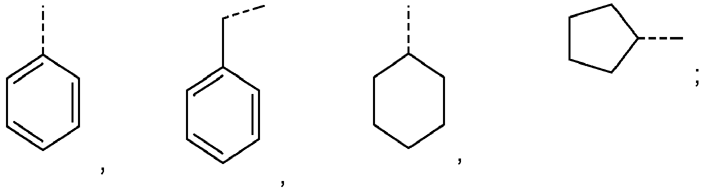 n and m are independently of each other selected from 1, 2, 3.
and enantiomers, stereoisomeric forms, hydrates, solvates, acid salt forms, tautomers, and racemates of the above mentioned compounds and pharmaceutically acceptable salts thereof for use in the therapeutic activation of autophagy.

Preferably R² represents hydrogen. Also preferably R¹ and R² represent hydrogen.

Preferably R¹ and R² are independently of each other selected from -R⁷, -R⁸, -COR⁷, -COR⁸, -CO₂R⁷, and -CO₂R⁸. More preferably R¹ and R² are independently of each other selected from -COR⁷ and -COR⁸.

Still more preferably R² represents hydrogen and R¹ represents -R⁷, -COR⁷, or -CO₂R⁷. Still more preferably R² represents hydrogen and R¹ represents -R⁷ or -COR⁷. Still more preferably R² represents hydrogen and R¹ represents -COR⁷ or -CO₂R⁷. Still more preferably R² represents hydrogen and R¹ represents -COR⁷.

Preferably R³ is selected from -H, -CH₃, -C₂H₅, -C₃H₇, -C₄H₉, or -C₅H₁₁; and more preferably from -H, -CH₃, -C₂H₅, or -C₃H₇; and most preferably from -H or -CH₃.

Preferably R⁴ represents H or R⁴ and R⁵ form together with the two aromatic carbon atoms they are connected to a cycle selected from: 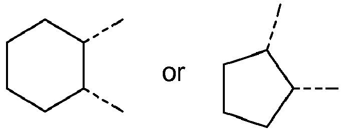 and most preferably from

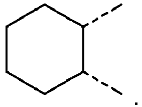

Preferably R⁵ is selected from: -CH₃, -C₂H₅, -C₃H₇, -C₄H₉, -C₅H₁₁, -C₆H₁₃, -C₇H₁₅, -C₈H₁₇, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH=C(CH₃)₂, -C=CH, -C≡C-CH₃, -CH₂-C≡CH, -C₂H₄-C≡CH, -CH₂-C≡C-CH₃, or -C≡C-C₂H₅, and more preferably from -CH₃, -C₂H₅, -C₃H₇, -C₄H₉, -C₅H₁₁, -C₆H₁₃, -CH=CH₂, -CH₂-CH=CH₂, -C(CH₃)=CH₂, -CH=CH-CH₃, -C=CH, -C≡C-CH₃, or -CH₂-C≡CH; and still more preferably from -CH₃, -C₂H₅, -C₃H₇, -C₄H₉, -C₅H₁₁, -CH=CH₂, -CH₂-CH=CH₂, or -C≡CH; and most preferably from -CH₃, -C₂H₅, -C₃H₇, -C₄H₉, or -C₅H₁₁.

In regard to the present invention *N*-(4-butyl-2-methylphenyl)acetamide (SMIP004) or 4-butyl-2-methylaniline (SMIP004-7) are the most preferred compounds according to the general formula I.

The structural chemical formula of SMIP004 is as follows:

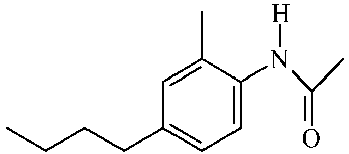

The structural chemical formula of SMIP004-7 is as follows:

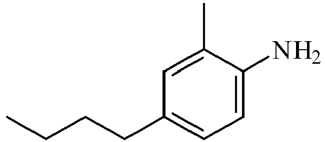

One aspect of the present invention is that the compounds of the general formula I, and in particular *N*-(4-butyl-2-methylphenyl)acetamide and N4-butyl-2-methylaniline are suitable for use in the treatment of a psychiatric disorder, infectious disease, a myopathy or a neurodegenerative disease. In other words the present invention refers to the use of a compound of the general formula I for the manufacture of a medicament for use in the treatment of a psychiatric disorder, infectious disease, a myopathy or a neurodegenerative disease. One aspect of the present invention refers to a method of treating a psychiatric disorder, infectious disease, a myopathy or a neurodegenerative disease comprising: administering to a subject who would benefit from such a treatment a therapeutically effective amount of a composition comprising a compound of the general formula I and optionally one or more pharmaceutically acceptable carriers.

One preferred embodiment of the present invention is a compound of general formula I for use in the treatment of a psychiatric disorder wherein the psychiatric disorder is an affective disorder (also called mood disorder), schizophrenia or a sleep disorder.

The affective disorder according to the invention is preferably selected from the group comprising or consisting of depressive disorder (such as major depressive disorder (MDD); also known as clinical depression or major depression), anxiety disorder, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is preferably selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

The neurodegenerative disorder according to the invention is preferably selected from the group comprising or consisting of Alzheimer's disease, Parkinson's disease, Machado-Joseph disease and Huntington's disease.

Autophagy is being recognized as an essential component of innate immunity; i.e., the elimination of pathogenic microorganisms (s. Gomes et al. Autophagy in Antimicrobial Immunity; Molecular Cell 2014, Volume 54(2), 224-233 describing several viruses and pathogens inhibited by autophagy). Therapeutic targeting of bacteria by means of autophagy induction is therefore being suggested as a useful strategy to combat intracellular infections (see Levine et al. Nature 2011, volume 469; 323 - 335). Listeria monocytogenes (L. monocytogenes) is a facultative Gram-positive bacteria and an intracellular pathogen that causes listeriosis. Py et al. reported (Autophagy. 2007; 3(2):117-25) that early *L. monocytogenes* growth is accelerated in autophagy deficient cells, compared to wild-type cells, suggesting an essential role for autophagy in inhibition of bacterial growth inside cells.

The inventors present herein examples showing that Skp2 inhibitors that induce autophagy protect cells from cell death after viral infection with corona viruses (Figs 18 and 19). Skp2 inhibitors also protect cells when applied at the time of viral infection (Fig. 20). In addition, the anti-viral role for autophagy during infection of various viruses has been described in the literature. Liang et al (J Virol 1998;72(11):8586-8596) describe that enforced expression of Beclin1 in mouse brains during Sindbis virus infection reduced viral titres and virus-induced neuronal apoptosis and thereby protects mice against Sindbis virus encephalitis. Campbell and Spector (Current Opinion in Microbiology 2013, 16:349-354) summarize studies demonstrating that inducers of autophagy inhibit HIV replication.

Therefore, one preferred embodiment of the present invention is a compound of general formula I for use in the treatment of an infectious disease wherein the infectious disease is a viral infection. It is especially preferred that the viral infection is a corona virus infection, such as infection with the Severe acute respiratory syndrome coronavirus (SARS-CoV) or Middle East respiratory syndrome coronavirus (MERS-CoV). The MERS-CoV was first reported and isolated in 2012. Most people who have been confirmed to have MERS-CoV infection developed severe acute respiratory illness. They had fever, cough, and shortness of breath. About 30% of the people confirmed to have MERS-CoV infection have died. Medical care is provided to support and relieve the signs and symptoms of MERS. However, there are no treatments available to cure the infection.

Studies in model organisms, including plants and Drosophila, have also confirmed an important role for autophagy/xenophagic genes in defence against two single-stranded RNA (ssRNA) viruses, tobacco mosaic virus (TMV) and vesicular stomatitis virus (VSV), suggesting that the anti-viral function of autophagy is evolutionarily conserved.

Therefore, another aspect of the present invention is directed to pharmaceutical compositions comprising at least one compound of the general formula I as active ingredient, together with at least one pharmaceutically acceptable carrier, excipient and/or diluents or solvents for use in the treatment of a psychiatric disorder, infectious disease, a myopathy or a neurodegenerative disease.

The pharmaceutical compositions of the present invention can be prepared in a conventional solid or liquid carrier or diluent at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to general formula I, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimize the therapeutic effect(s), e.g. antidepressant activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticizers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilized in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The pharmaceutical compositions may further comprise at least one further active agent. It is preferred if this active agent is selected from the group consisting of anti-depressant and other psychotropic drugs. It is further preferred if the anti-depressant is selected from amitriptyline, amioxide clomipramine, doxepine, duloxetine, imipramine trimipramine, mirtazapine, reboxetine, citalopram, fluoxetine, moclobemide and sertraline.

Another aspect of the present invention relates to screening methods for novel Skp2 inhibitors and/or Beclin1 stabilizers that should be suitable as antidepressant.

Therefore, one preferred embodiment of the present invention refers to a method for screening for a compound for use in the therapeutic activation of autophagy and especially for the treatment of a psychiatric disease, infectious disease, a myopathy or a neurodegenerative disease, wherein the method comprises the following steps:
a) contacting a test compound (or candidate substance) with at least one polypeptide selected from the group consisting of Skp2, Beclin1 and Akt polypeptide,
b) detecting the binding of said test compound to the selected polypeptide, and
c) determining the activity or stability of the at least one polypeptide of step a) in the presence of said test compound.

An especially preferred embodiment of the present invention refers to a method for screening for a compound for use in the therapeutic activation of autophagy and especially for the treatment of a psychiatric disease, infectious disease, a myopathy or a neurodegenerative disease, wherein the method comprises the following steps:
a) contacting a test compound (or candidate substance) with at least Skp2,
b) detecting the binding of said test compound to Skp2, and
c) determining the total levels of Beclin1 in the presence of said test compound.

The test compound to be tested within a method of the present invention for use in the therapeutic activation of autophagy may be each chemical molecule. It is preferred if the test compound is a so called small molecule. The term small molecule refers to low molecular weight organic compound which is by definition not a polymer. In the field of pharmacology, it is usually restricted to a molecule that also binds with high affinity to a biopolymer such as proteins, nucleic acids, or polysaccharides. Small molecules are broadly used as enzyme inhibitors, thus they are preferred compounds for screening for inhibitors of Skp2 or compounds interfering with stability of Beclin1, preferably for compounds stabilizing Beclin1 (in particular by decreasing ubiquitination of Beclin1). It is further preferred if the test compound is a polypeptide like a recombinant protein, a biopharmaceutical, also known as a biological or a biosimilar.

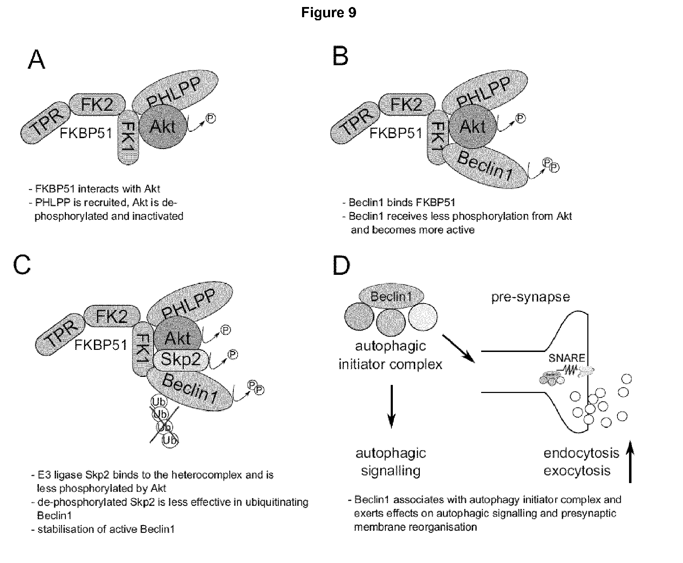

Preferably, the test compound in solid form or a solution of the test compound may be added. Thereby, it is preferred that different concentrations of the test compound will be added in a way that a concentration series is formed. The evaluation of a concentration series may allow calculating the affinity of the test compound for binding to Skp2, Beclin1 or Akt polypeptide. The ideal substance would be a compound inhibiting Skp2 and/or enhancing stability of Beclin1. The phosphorylation of Skp2 can be used as an indicator for the activity or respectively inhibition of Skp2 (cf. Fig. 9). The ubiquitination of Beclin1 or the protein level of Beclin1 can be determined and used to conclude on the stability of Beclin1. Step c) determining the activity or stability of the selected polypeptide in the presence of said test compound could also comprise determining the amount of FKBP51-Beclin1 complex formation. Another read out suitable within said method of the invention could be the lipidation of LC3B-1 (yielding LC3B-II). Also Evaluation of the amount of the formation of autophagosomes in response to the test compound is suitable. Hereby, an increase of FKBP51-Beclin1 complex formation and autophagosome formation is indicative for an antidepressant effect of the test compound.

Additionally, it is possible to add not only the test compound but also another component, which should be analyzed in regard to its influence on the effect of the test compound on the binding to one of the mentioned polypeptides and thus, on the treatment of said diseases.

As used herein "contacting" means bringing the test compound (or candidate substance) and the at least one polypeptide in contact, namely under time and temperature conditions sufficient for the test compound to bind to said polypeptide. In this respect, contacting may also be referred to "incubating". The term polypeptide refers to al natural occurring forms of polypeptides, such as phosphorylated or dephosphorylated polypeptide.

Step a) of the inventive method could also be defined as mixing the test compound with the at least one polypeptide. Step a) as well as the complete method of the invention could be performed in solution. This means that the test compound and the at least one polypeptide may be solved together in one solution or that a solution of the at least one polypeptide is mixed with a solution of a test compound. Such a solution could be based on any suitable solvent as well as buffer solutions or a mixture of a solvent, especially an organic solvent and a buffer. It should be avoided that the solvent or buffer denatures the polypeptide. Hence, denaturants, surfactants or other amphiphilic molecules in the solution should be avoided.

Alternatively, the inventive method could be performed in cultured or isolated cells, such as in PBMCs. Then step a) of the inventive method would comprise adding the test compound to the cell medium and the activity or stability of the selected polypeptide could be determined in a lysates of the cells treated with the test compound. Alternatively, formation of autophagosomes could be detected by immunofluorescence staining and subsequently light microscopy.

A test compound in regard to the present invention, such as for example siRNA, could also interact with the expression rate of Skp2 or Akt. Therefore, the present invention refers also to a method, wherein instead of polypeptides, nucleic acids encoding the polypeptides are used and the expression rate is determined instead of the activity.

Thus, another aspect of the present invention refers to a method for screening for a compound for use in the therapeutic activation of autophagy and especially for the treatment of a psychiatric disease, infectious disease, a myopathy or a neurodegenerative disease, wherein the method comprises the following steps:
a) contacting a test compound (or candidate substance) with at least one nucleic acid encoding a polypeptide selected from the group consisting of Skp2, Beclin1 and Akt polypeptide,
b) detecting the binding of said test compound to the selected nucleic acid, and
c) determining the expression rate of the respective polypeptide in the presence of said test compound.

The expression level of genes can be determined on the nucleic acid level, preferably on the level of mRNA. Levels of mRNA can be quantitatively measured by Northern blotting. A sample of RNA is separated on an agarose gel and hybridized to a labeled RNA probe that is complementary to the target sequence. The labeled RNA is then detected. Alternatively the level of mRNA can be measured by reverse transcription quantitative polymerase chain reaction (RT-PCR followed with qPCR) to compare mRNA abundance between samples, e.g. between a cancer and a non-malignant cell sample.

There are several types of depression drugs (antidepressants) used to treat depression and conditions that have depression as a component of the disease, including selective serotonin reuptake inhibitors (SSRIs), atypical antidepressants, tricyclic antidepressants (TCAs), and monoamine oxidase inhibitors (MAOIs). However, research shows that antidepressants fall short for many people. Finding the right drug and dosage is a trial and error process which may take a long time. It takes approximately four to six weeks for antidepressant medications to reach their full therapeutic effect. Furthermore to stop taking an antidepressant, it is advised to taper off slowly. Many people try several medications before finding one that helps. Therefore there is a need for a method that could help determining if a subject suffering from depression responds to a specific antidepressant, before starting therapy. The inventors could demonstrate that in human Peripheral Blood Mononuclear Cells, FKBP51 levels correlate with the potential of antidepressants to induce autophagic pathways. Further, data from an inpatient sample of individuals suffering from depression, show that the levels of FKBP51, FKBP51-directed proteins and Beclin1 in peripheral blood cells at admission correlate with the antidepressant treatment response. Thus, the antidepressant response of autophagic markers in *ex vivo* cultivated blood cells from inpatients predicts the clinical antidepressant response. In particular the response of Peripheral Blood Mononuclear Cells in culture to antidepressants, determined by the expression of Beclin1, FKBP51, phosphorylated Akt (pAkt^{S473}), phosphorylated DNA methyltransferase 1 (pDNMT1^{S154}), phosphorylated Gsk3β (pGsk3β^{S9}) and Atg12 can be predictive for the success of antidepressants in the clinic.

Therefore, the present invention relates to a method of determining if a subject suffering from depression responds to an antidepressant, comprising:
measuring the protein level of at least one of FKBP51, Beclin1, pAkt^{S473}, pAkt^{S473}/Akt, phosphorylated tau (ptau^{S622}), pGsk3βS9, pGsk3βS9/Gsk3β and Atg12 in a blood sample from said subject comprising peripheral blood mononuclear cells from said subject or in isolated peripheral blood mononuclear cells from said subject, wherein at least one increased protein level of the group comprising or consisting of: FKBP51, Beclin1, pGsk3βS9, pGsk3βS9/Gsk3β and Atg12 and at least one decreased protein level of pAkt^{S473} or pAkt^{S473}/Akt and ptau^{S622} is indicative for a response to the antidepressant.

It is sufficient to measure or determine the protein level of only one of FKBP51, Beclin1, pAktS473, pAktS473/Akt, ptau^{S622}, pGsk3β^{S9}, pGsk3β^{S9}/Gsk3β and Atg12. However, determination of two levels may further increase the significance of the method. Consequently, in a further preferred embodiment of the invention, two of the peptide levels are determined or measured in combination. The possible combinations are FKBP51 + Beclin1, FKBP51 + pAkt^{S473}/Akt, FKBP51 + pGsk3β^{S9}/Gsk3β, FKBP51 + Atg12, Beclin1 + pAkt^{S473}/Akt, Beclin1 + pGsk3β^{S9}/Gsk3β, Beclin1 + Atg12, pAkt^{S473}/Akt + pGsk3β^{S9}/Gsk3β, pAkt^{S473}/Akt + Atg12, pGsk3β^{S9}/Gsk3β + Atg12, FKBP51 + ptau^{S622}, Atg12 + ptau^{S622}, pAkt^{S473} + ptau^{S622}, Beclin1 + ptau^{S622}, pAkt^{S473}/Akt + ptau^{S622} and pGsk3β^{S9}/Gsk3β + ptau^{S622}. In a further preferred embodiment of the invention, at least three levels, such as FKBP51 + Beclin1 + pAkt^{S473}/Akt or FKBP51 + Beclin1 + Atg12 may be determined at once for an increased significance.

Thereby the level of FKBP51, Beclin1, pGsk3β^{S9}, pGsk3β^{S9}/Gsk3β, ptau^{S622}, Atg12 and of pAkt^{S473}, pAktS473/Akt can be compared to standard values or normal ranges. Thereby the term "normal range" is defined as the range of protein levels seen in 95 percent of healthy people in a certain group. Thereby, the group may depend of the age, gender and race of the patient. Thus, an alternative wording of the above method reads as follows:
A method of determining if a subject suffering from depression responds to an antidepressant, comprising:
   measuring the protein level of at least one of FKBP51, Beclin1, ptau^{S622}, pAktS473, pAktS473/Akt, pGsk3β^{S9}, pGsk3β^{S9}/Gsk3β and Atg12
   in a blood sample from said subject comprising peripheral blood mononuclear cells from said subject or in isolated peripheral blood mononuclear cells from said subject, wherein at least one increased protein level of FKBP51, Beclin1, pGsk3β^{S9}, pGsk3β^{S9}/Gsk3β, Atg12 or a decreased level of pAkt^{S473}, pAkt^{S473}/Akt or ptau^{S622} compared to a normal range for the respective protein level, is indicative for a response to the antidepressant.

The effect of different antidepressants on a specific subject or patient can also be determined by treating isolated peripheral blood mononuclear cells from said subject with the antidepressants and evaluating FKBP51, Beclin1, pAkt^{S473}, pAkt^{S473}/Akt pDNMT1, pGsk3β^{S9}, pGsk3β^{S9}/Gsk3β and Atg12 as biomarker for an antidepressant effect.

One preferred embodiment of the present invention relates therefore to a method for determining if a subject suffering from depression will respond to an antidepressant, comprising:
measuring the protein level of at least one of FKBP51, Beclin1, pDNMT1^{S154}/DNMT1, pAkt^{S473}/Akt, pGsk3β^{S9}/Gsk3β and Atg12
in peripheral blood mononuclear cells from a blood sample from said subject after incubation of the cells with the antidepressant (preferably also in peripheral blood mononuclear cells without the antidepressant),
wherein at least one increased protein level of FKBP51, Beclin1, pGsk3β^{S9}/Gsk3β and Atg12 or at least one decreased protein level of pAkt^{S473}/Akt and pDNMT1 in the sample with the antidepressant, preferably compared to the cells without the antidepressant, is indicative for a response to the antidepressant.

Again, it is sufficient to measure or determine the protein level of only one of FKBP51, Beclin1, pAkt^{S473}/Akt, pDNMT1^{S154}/DNMT1, pGsk3β^{S9}/Gsk3β and Atg12. However, determination of two levels may further increase the significance of the method. Consequently, in a further preferred embodiment of the invention, two of the peptide levels are determined or measured in combination. The possible combinations are FKBP51 + Beclin1, FKBP51 + pAkt^{S473}/Akt, FKBP51 + pGsk3β^{S9}/Gsk3β, FKBP51 + Atg12, Beclin1 + pAkt^{S473}/Akt, Beclin1 + pGsk3β^{S9}/Gsk3β, Beclin1 + Atg12, pAkt^{S473}/Akt + pGsk3β^{S9}/Gsk3β, pAkt^{S473}/Akt + Atg12, pGsk3β^{S9}/Gsk3β + Atg12, pDNMT1^{S154}/DNMT1 + Atg12, pDNMT1^{S154}/DNMT1 +/ Beclin1, pDNMT1^{S154}/DNMT1 + pGsk3β^{S9}/Gsk3β, pDNMT1^{S154}/DNMT1 + pAkt^{S473}/Akt. In a further preferred embodiment of the invention, at least three levels, such as FKBP51 + Beclin1 + pAkt^{S473}/Akt or FKBP51 + Beclin1 + Atg12 may be determined at once for an increased significance. In addition it is preferred to determine formation of a heterocomplex of Beclin1 with protein kinase Akt and the E3 ligase Skp2.

There is no need to say that a conclusion that an increase in protein level can only be determined in comparison between two levels. Suitable level can be compared between the sample after incubation with the antidepressant and the sample before incubation. Another possibility would be to compare protein level in the sample after incubation with the antidepressant and protein level in a sample from the same subject without incubation with the antidepressant (instead incubation with a control, such as solvent only, should be performed). The protein level in the sample after incubation with the antidepressant can also be compared to level in a sample from the same subject after incubation with another antidepressant.

The method according to the present invention is preferred if the protein level of at least one of FKBP51, Beclin1, pGsk3β^{S9}/Gsk3β and Atg12 is increased or of pAkt^{S473}/Akt is decreased in said cells after incubation at least 1.5-fold, preferred at least 2-fold and more preferably at least 4-fold compared to the level in the cells from said subject before incubation or without incubation, or respectively with incubation of solvent of the antidepressant only.

### Description of the Figures:

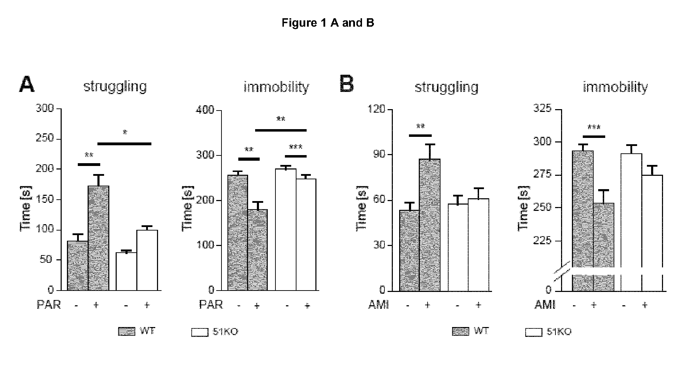

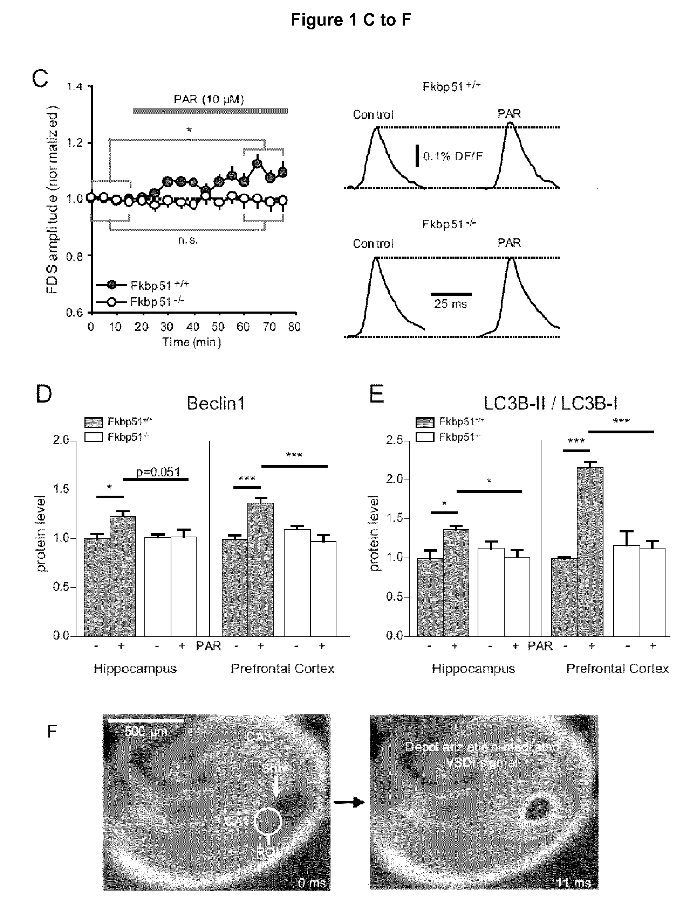

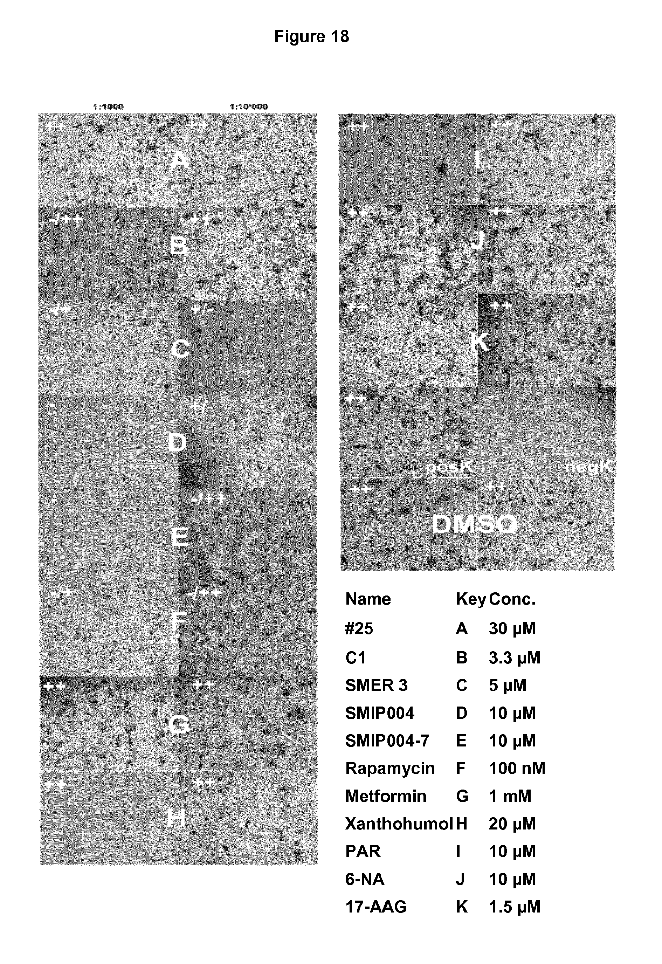

**Figure 1****.** *FKBP51 is required for the effects of PAR on behavior, on evoked CA1 neuronal activity, and on autophagic markers in mice.* A, FKBP51^{-/-} and FKBP51^{+/+} mice were treated with paroxetine (PAR 10 mgkg-1) or vehicle and subjected to the forced swim test (n=7-9 per group). Graphs show the times of immobility and struggling. B, a similar experiment was performed with amitriptyline (AMI 10 mgkg⁻¹) instead of PAR. C, Left, quantification of PAR's effects on CA1 FDSs (n=10 slices from 4 mice for each group). *p=0.032 (paired t-test); n.s., p=0.912 (paired t-test); data are given as mean ±SEM. Right, representative recording traces illustrating the PAR effects on CA1 FDSs. D,E, FKBP51-/- and FKBP51+/+ mice were treated with PAR (10 mgkg-1) or vehicle and sacrificed 45 mins later. Protein levels were determined in extracts of the hippocampus and the prefrontal cortex from 9-10 animals in technical triplicates. *p<0.05; **p<0.01; ***p<0.001. F, Left, illustration of the position of the stimulation electrode (Stim) and ROI used for the calculation of CA1 neuronal population activity. Right, depolarization-mediated VSDI signal recorded 11 ms after the electrical stimulation pulse. Darker grey represent higher values of the fractional change in fluorescence (dF/F) and, thus, stronger neuronal activity.

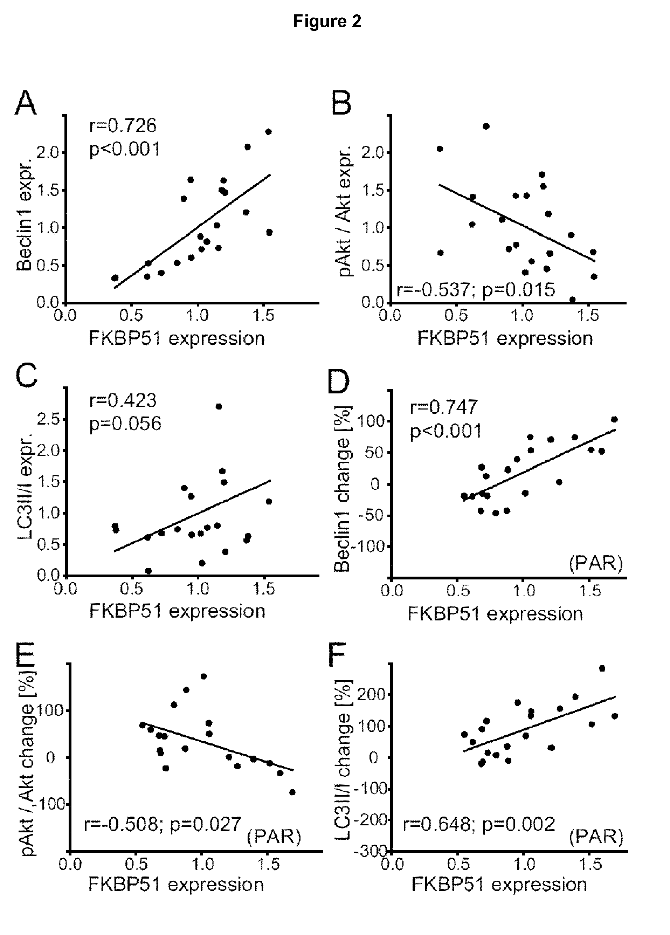

**Figure 2****.** *Correlation of FKBP51 with autophagy pathway components and with effects of ADs in human PBMCs*. A-C, protein levels of FKBP51, Beclin1, pAkt^{S473}/Akt, and LC3B-II/I, in PBMCs from healthy male subjects (n=21). Each dot represents the levels of FKBP51 and the respective protein in PBMCs from one individual subject. Average expression levels were set to 1. D-F, levels of FKBP51, Beclin1, pAkt^{S473}, and LC3B-II/I in PBMCs from healthy male subjects (n=20), cultivated *ex vivo* and treated with PAR (0.365 µM, 48 h). Plots depict protein changes upon treatment with AD compared to vehicle-treated cells in correlation to FKBP51. Each dot represents the level of FKBP51 and the expression change of the respective protein in PBMCs from one individual.

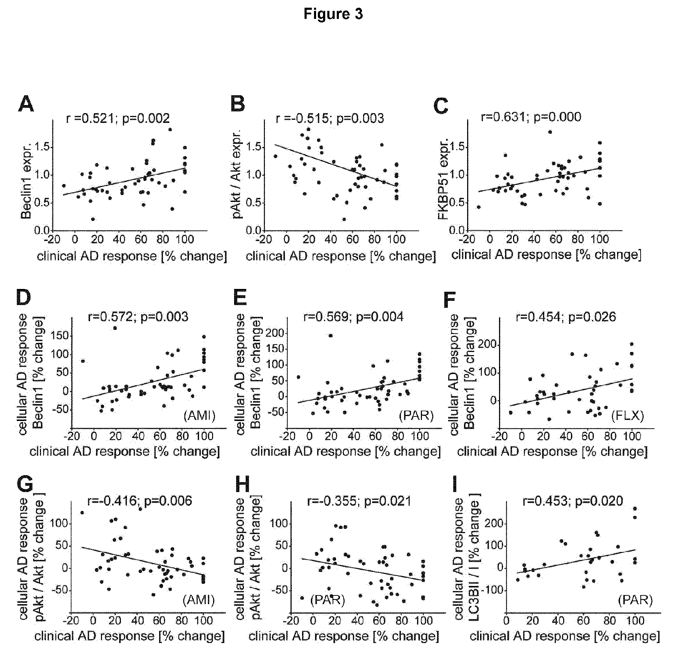

**Figure 3****.** *Correlation of clinical antidepressant response with the expression of FKBP51, pAkt and Beclin 1 and with the effects of antidepressants in PBMCs ex vivo.* Beclin1, FKBP51, LC3B-II/I and pAkt^{S473}/Akt levels were determined in PBMCs from inpatients with depression at admission (n=51). **A-C,** Expression values are correlated with the response to antidepressant-treatment expressed as percent change in the total score of the 21-items Hamilton Depression Rating scale between admission and after 6 weeks of treatment. **D-I,** PBMCs from depressive inpatients were cultivated *ex vivo* and treated with PAR (0.365 µM), FLX (1695 nM) or AMI (888 nM) for 48 h. The antidepressant induced changes in expression of Beclin1, pAkt^{S473}/Akt and LC3B-II/I are correlated with the clinical response to antidepressant-treatment expressed as percent change in the total score of the 21-items Hamilton Depression Rating scale between admission and after 6 weeks of treatment (D,E,G,H: n=51; F: n=43; I: n=31).

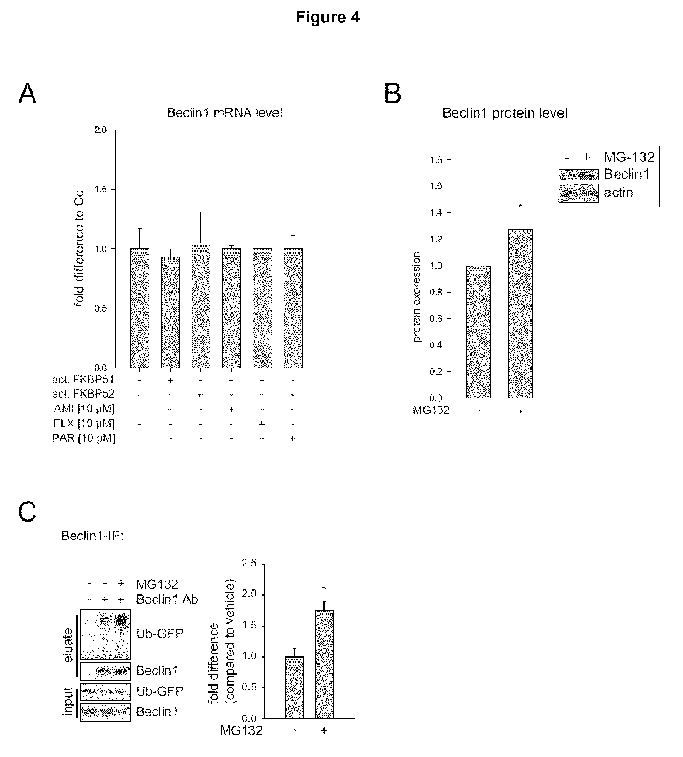

**Figure 4****.** *Regulation of Beclin1 protein level by ubiquitination. (A) FKBPs and antidepressants do not change Beclin1-mRNA (but protein, as shown in* *figure 1**).* Rat cortical astrocytes were transfected with expression vectors encoding for FKBP51 or FKBP52, or were stimulated with amitriptyline (AMI, 10µM), fluoxetine (FLX, 10µM) or paroxetine (PAR, 10µM) for 72h. Beclin1 mRNA expression levels determined by qPCR. Vector transfected and vehicle stimulated mRNA expression was set to 1. (B) Beclin1 protein homeostasis depends on the UPS (ubiquitin proteasome system). HEK cells were treated either with proteasome inhibitor MG132 or with vehicle for 5h. Beclin1 protein levels were determined by Western analysis. (C) Beclin1 is ubiquitinated. Immunoprecipitations were performed with Beclin1 antibody in lysates from HEK cell ectopically expressing Ub-GFP. Cells had been treated with proteasomal inhibitor MG132 or vehicle respectively. Ubiquitination was detected using a GFP antibody. Ub binding under vehicle treated conditions was set to 1. Bars represent relative mean protein expression +SEM (n=3). *p<0.05.

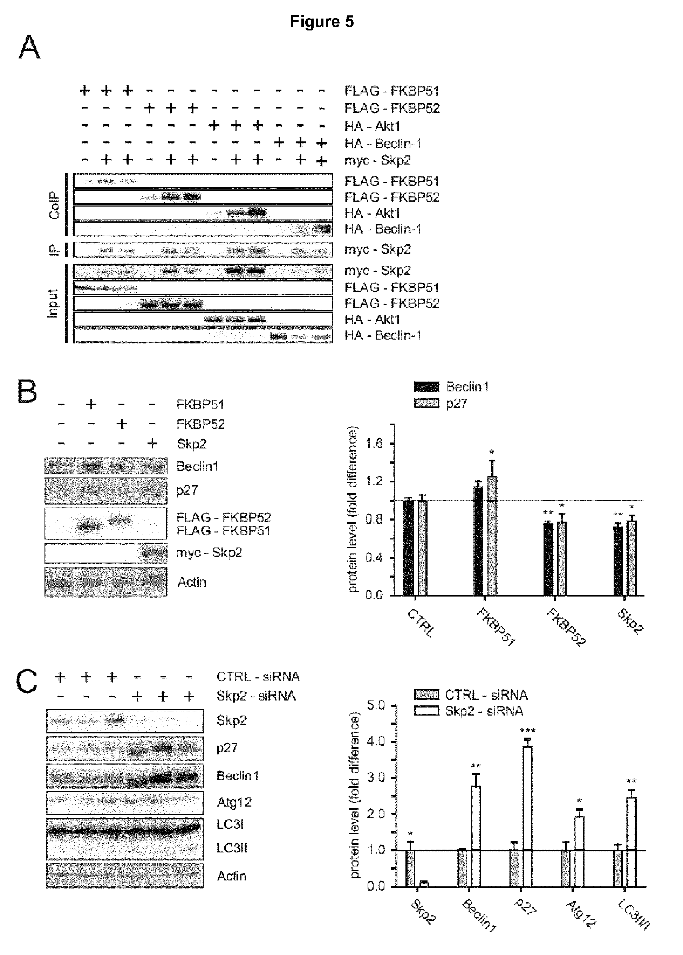

**Figure 5****.** *Functional interaction of Skp2 with Beclin1, Akt, and FKBP51.* HEK cells were transfected with plasmids encoding FLAG-tagged FKBP51/52, HA-tagged Beclin1 or Akt1 and myc-tagged Skp2 constructs. After precipitation of protein complexes using a myc-antibody, (co)precipitated proteins were quantified. (A) Representative Western blots of the interaction analyses. (B) Effects of ectopic expression of tagged proteins from (A) on Beclin1 and p27 protein levels. left, representative Western blots. Right, graphs show the relative mean expression +SEM (n=3). Protein levels of vector transfected HEK cells were set to 1. (C) Genetic knock-down of Skp2 by siRNA induces Beclin1 and markers of autophagy. left, representative Western blots. p27 serves as a control for Skp2 activity. Right, bars represent relative mean protein expression +SEM (n=3). *p<0.05; **p<0.01; ***p<0.001.

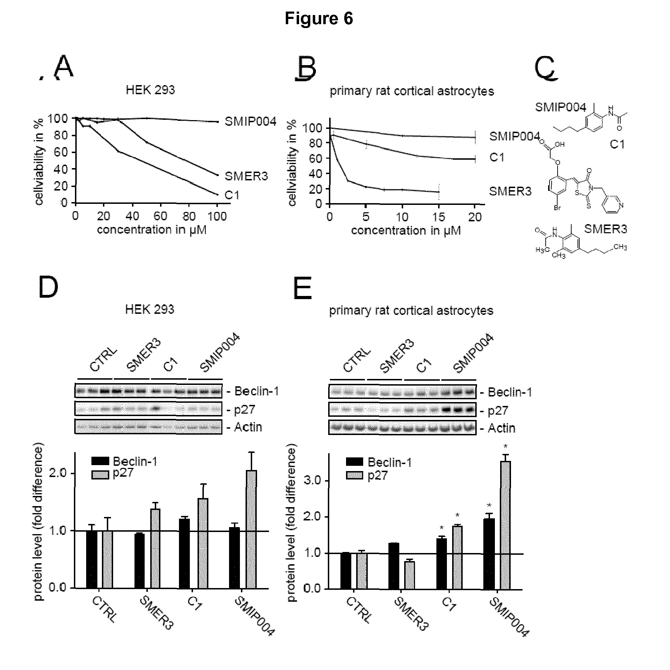

**Figure 6****.** *Testing small compounds inhibiting Skp2: cell toxicity and Beclin1 stabilization.*
   Comparison of three small molecule inhibitors on cytotoxicity determined by an MTT assay performed in (A) HEK 293 cell and in (B) primary rat cortical astrocytes. Graphs display the mean ±SEM difference to control (vehicle) in triplicates. Vehicle was set to 100%. (C) Chemical structures of the analyzed molecules. Effects of small compounds on Beclin1 and p27 protein levels determined by Western analysis of (D) HEK 293 (E) primary rat cortical astrocytic lysates. Bars represent the mean +SEM (n=3). *p<0.05; **p<0.01; ***p<0.001.

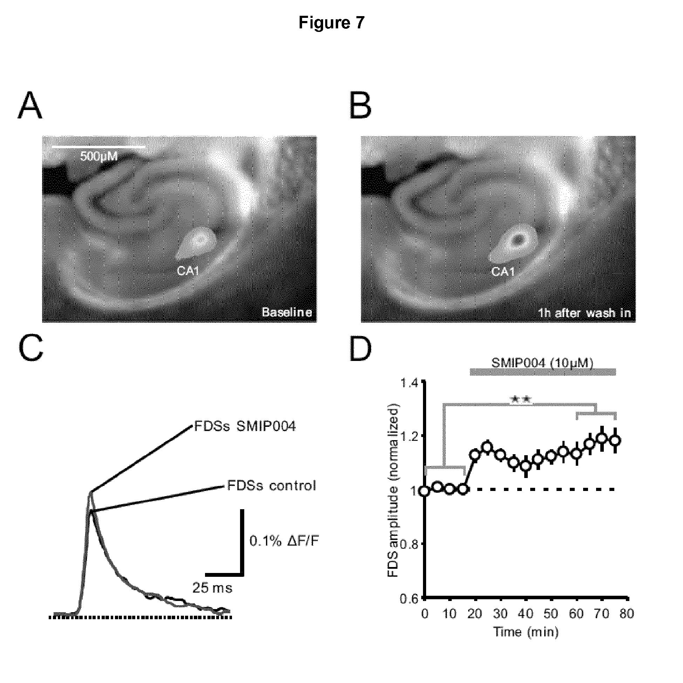

**Figure 7****.** *Inhibition of Skp2 by SMIP004 elicits antidepressant-like effects by enhancing evoked CA1 neuronal activity.*
   Depolarization-mediated VSDI (voltage-sensitive dye imaging) signal recorded (A) at baseline conditions (B) 1 h after wash in of 10 µM SMIP004. Warmer colors represent higher values of the fractional change in fluorescence (ΔF/F) and, thus, stronger neuronal activity. (C) Quantification of SMIP004's effects on CA1 FDSs (fast depolarization-mediated signal; n=9 slices from 6 C57BL/6 mice). **p=0.004 (paired t-test); data are given as mean ±SEM. (D) representative recording traces illustrating the SMIP004 effects on CA1 FDSs.

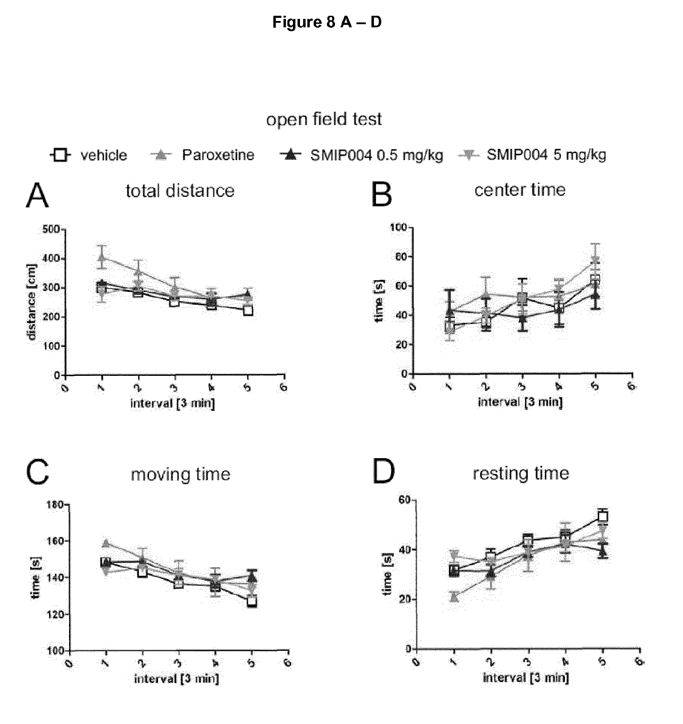

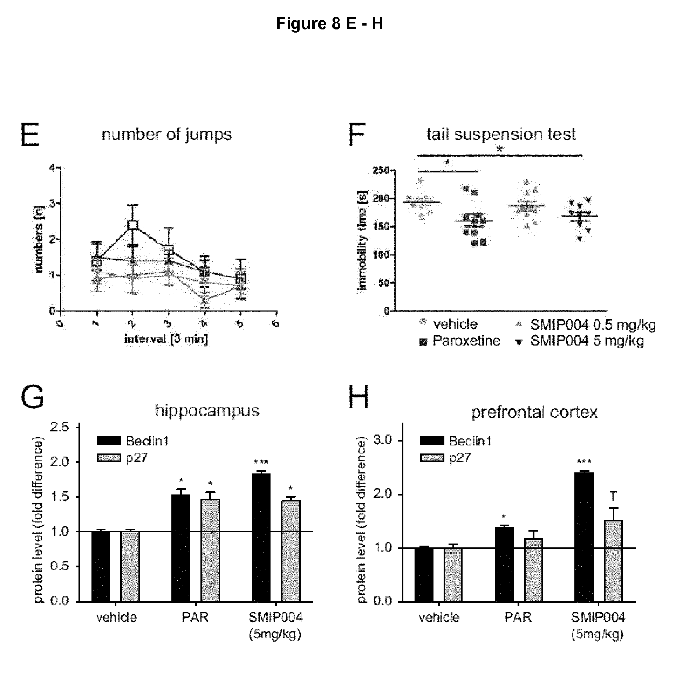

**Figure 8****.** *Pharmacological inhibition of Skp2 by SMIP004 causes antidepressant-like behavior in mice.*
   C57BL/6 mice were treated with SMIP004 (0.5 mgkg-1 or 5 mgkg-1), paroxetine (10 mgkg-1) or vehicle and subjected to the open field test (A-E) and the tail suspension test (F; n=10 per group). Mice were sacrificed after behavioral testing. Protein levels were determined in extracts of the (G) hippocampus and the (H) prefrontal cortex. Curves/bars represent the mean ±SEM. T<0.1; *p<0.05; **p<0.01; ***p<0.001.
**Figure 9****.** *Model of the Mechanism of FKBP51's impact on the Akt-Beclin1 heterocomplex and its pharmacological modulation.*
   Through its PPlase domain (FK1), FKBP51 interacts with PHLPP, Akt, and Beclin1, thereby recruiting inactive Akt (via PHLPP-mediated de-phosphorylation at S473) to Beclin1 (A, B). The resulting dephosphorylated Beclin1 induces autophagic pathways. Additionally, FKBP51-mediated inhibition of Akt leads to inactivation of Skp2 E3 ligase (via inhibition of Akt-mediated Skp2 phosphorylation at S72) and thus suppresses the degradative effect by ubiquitination on Beclin1 (C). Active Beclin1 associates to the autophagy initiator complex that triggers autophagic signalling (D). Beclin1 not only affects autophagic signalling, but also synaptic neurotransmission. SMIP004 directly inhibits Skp2 E3 ligase, independent of the Akt or FKBP51 status and thereby enhances Beclin1 protein level. ADs act on the same pathways in an FKBP51-dependent manner and change FKBP51 protein interactions; this could form the basis for the FKBP51-dependency of AD effects in cells, animals, and humans.

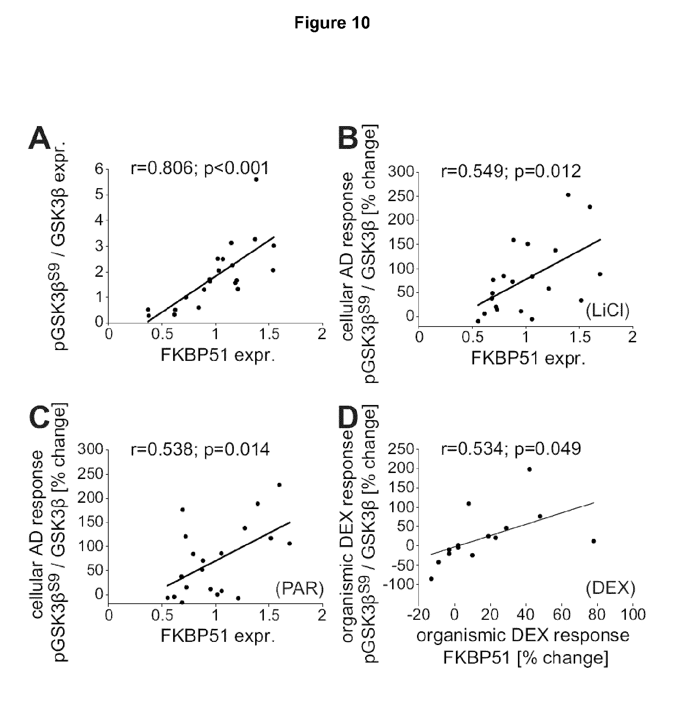

**Figure 10****.** *FKBP51 in human peripheral blood lymphocytes correlates with pGSK3*β *and the effects of lithium, PAR and Dex.*
   A-C, PBMCs were collected from healthy male subjects (N=21) and protein levels were determined in cell extracts by Western blotting (A). PBMCs were also cultivated *ex vivo* and treated with lithium (190 µM), PAR (365 nM) or vehicle for 48 h and protein levels were determined in cell extracts by Western blotting. Change of pGSK3β^{S9}/GSK3β was calculated from the comparison of drug treated cells with vehicle treated cells. Each dot represents the levels of the respective proteins in PBMCs from one individual. Average expression levels of FKBP51 were set to 1. D, 16 subjects received 1.5 mg DEX and protein changes in PBMCs were determined 6 h later.

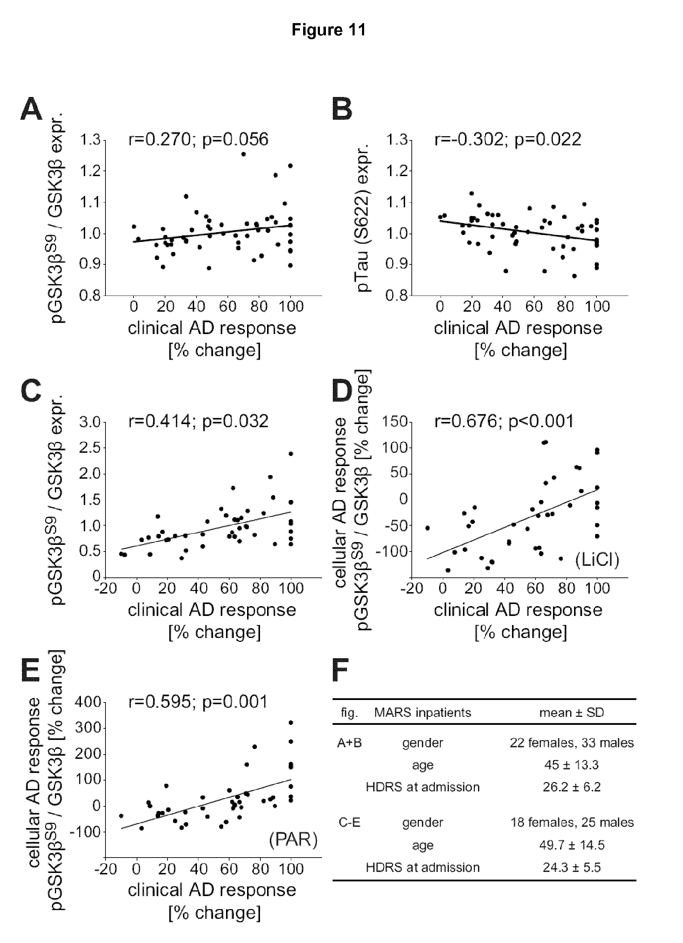

**Figure 11****.** *Markers of GSK3*β *activity in PBMCs of depressive patients and the cellular response to psychoactive drugs predict clinical improvement*.
   A, B, PBMCs were collected from depressive patients at the day of admission to the clinic (N=54). Protein concentrations in extracts were determined on a protein array and correlated to the change in Hamilton Depression Score. C-E, PBMCs were isolated from another cohort of patients at the day of admission to the clinic (N=43) and the pGSK3β^{S9} levels were determined by Western blotting (C). Part of the PBMCs were cultivated *ex vivo* and treated with lithium (190 µM), PAR (365 nM) or vehicle before determining the pGSK3β^{S9} levels by Western blotting (D, E). The levels of pGSK3β^{S9} (C) and the cellular change of pGSK3β^{S9}/GSK3β (D, E) was correlated to the change in Hamilton Depression Score.
**Figure 12****.** Aβ42 was determined using an ELISA assay in plasma and CSF from Alzheimer model mice treated with SMIP004 or vehicle.
**Figure 13****.**
   APP-CTF was determined by Western blot analysis in brain extracts from Alzheimer model mice treated with SMIP004 or vehicle.

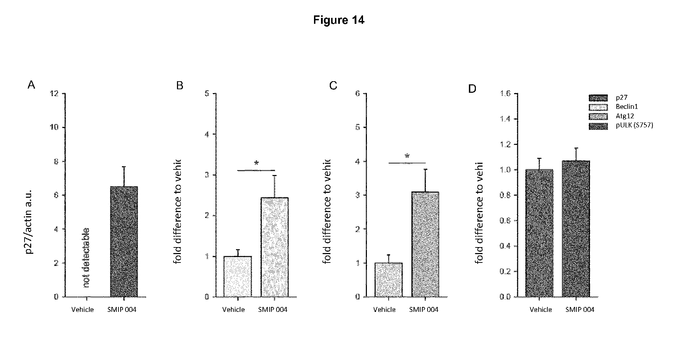

**Figure 14****.** The levels of p27 (target of Skp2), *Beclin1,* Atg12 and pULK^{S757} were determined by Western blotting in brain extracts of Alzheimer model mice treated with SMIP004 or vehicle.
**Figure 15****.**
   Beclin1 was immunoprecipitated from brain extracts of Alzheimer model mice treated with SMIP004 or vehicle. The levels of ubiquitinylation (as indicator of Skp2 inhibition) and of association with Vps34 (indication of autophagy initiation) was determined by Western blots in eluates of the immunoprecipitation.
**Figure 16****.**
   VeroB4 cells were treated for 72h with various compounds reported to inhibit Skp2. as Skp2 inhibitor, Cell viability was assessed with the MTT assay. Similar results were obtained in CACO2 cells.
**Figure 17****.**
   CACO2 cells were treated with the indicated compounds (for concentrations see figure 18) for 72h and protein ex-tracts were assessed for the levels of the autophagy markers Beclin1 and LC3II/I by Western blotting.
**Figure 18****.**
   VeroB4 cells were pretreated for 48h with putative or known autophagy inducers, infected with virus (MERS) and analyzed 48h thereafter (continuous drug treatment). Compounds were used at the concentrations given on the right, and also at 10 fold lower concentrations. Representative light microscopy images are displayed.
**Figure 19****.**
   VeroB4 cells were treated and infected with virus as in figure 18. 24h and 48h after infection, the amount of viral RNA was determined by qPCR. The designations "compound high" and "compound low" refer to the concentrations listed in figure legend 18 and 10 fold dilutions, respectively.
**Figure 20****.**
   VeroB4 cells were infected with MERS virus and treated with compound. Compound key as in figure 18. 24h, 48h and 72h after infection, the amount of viral RNA was determined by qPCR. The designations "compound high" and "compound low" refer to the concentrations listed in figure legend 18 and 10 fold dilutions, respectively.
**Figure 21****.**
   **A**, Time course of the experiment. Body weight was assessed on day 1, day 22 and day 43. Basal corticosterone was measured on day 22 and day 43. The behavioral testing (open field, social avoidance, forced swim test) and the stress response test (corticosterone samples 30 min and 90 after the FST) was performed in the last week of the treatment phase. **B-G**, weights of body, thymus and adrenal glands. When the three-way ANOVA indicated an interaction effect (p < 0.1), genotype-dependent stress and treatment effects were isolated by normalizing the data to either non-stressed controls or vehicle treatment. **B,** Body weight was significantly reduced in 51 KO mice before the start of the first defeat; CSDS induced a significant increase of body weight, while 51 KO mice showed still significantly reduced body weight, independent of condition. **C,D,** Stress-induced body weight increase was still present after 3 weeks of recovery. PAR-induced increase of body weight was most pronounced under control conditions. 51 KOmice were less affected by paroxetine-induced body weight increase. **E,F,** 51 KO mice showed overall reduced adrenal gland weight compared to wild-type mice. Adrenal gland weight was significantly increased in stressed animals. The stress effect was less pronounced in 51 KOmice compared to wild-type animals. Paroxetine led to adrenal gland enlargement in WT and reduced size in 51KOmice. **G**, Thymus size was significantly reduced following CSDS. 51KOmice demonstrated significantly lower thymus weight than wild-type mice. # main genotype effect, * main condition effect, $ main treatment effect, *a* genotype x treatment interaction, *b* genotype x condition interaction, *c* condition x treatment interaction; for #, *, $, a, b: p < 0.05; for c p < 0.1; data are expressed as mean + SEM.
**Figure 22****.**
   Time course is provided in Figure S13A. When the three-way ANOVA indicated an interaction effect (p < 0.1), genotype-dependent stress and treatment effects were isolated by normalizing the data to either non-stressed controls or vehicle treatment. **A,** CSDS resulted in significantly increased basal corticosterone levels in wild-type, but not in 51 KOmice. **B,** Basal corticosterone levels assessed three weeks after the stressor were significantly lower in 51KOmice. **C,** Basal corticosterone levels of 51 KOmice were less affected by the long lasting effects of CSDS. **D,** Paroxetine decreased basal corticosterone especially in 51 KOmice. **E** and **F,** Circulating corticosterone was significantly decreased in 51 KOmice in response to an acute stressor as well as after a 90 min recovery period. # main genotype effect, a genotype x treatment interaction, *b* genotype x condition interaction, § Tukey post-hoc; for #, b, §: p<0.05; for *a*, p < 0.1; data are expressed as mean + SEM.
**Figure 23****.**
   The left panel (pDnmt1 as state marker) shows the levels of Dnmt1 and of phosphorylated Dnmt1 (serine 154) determined in PBMCs from depressive inpatients at admission and 6 weeks after treatment in the clinic.
   The right panel (pDnmt1 as predictor) shows the PBMCs from depressive inpatients obtained at admission and treated with paroxetine or vehicle for 48 h.
**Figure 24****.**
   Wild-type and 51 KO mice were subjected to CSDS and subsequent chronic PAR treatment resulting in 8 experimental groups (n=8-12 per group) that were analyzed for behavior and autophagy marker protein levels. When the three-way ANOVA indicated an interaction effect (p < 0.1), genotype-dependent stress and treatment effects were isolated by normalizing the data to either non-stressed controls or vehicle treatment. **A,** General locomotion in the open field was independent of genotype, condition or treatment of the mice. **B - D,** deletion of FKBP51 abolished the reaction to CSDS and to chronic PAR treatment in the social avoidance test. **E-H,** deletion of FKBP51 significantly reduced the effect of chronic PAR treatment in the forced swim test. **I-R,** deletion of FKBP51 abolished the effects of chronic PAR treatment on the autophagy pathway markers Beclin1, Atg12, pAkt^{S473}/Akt and LC3BII/I. # main genotype effect, $ main treatment effect, *a* genotype x treatment interaction, *b* genotype x condition interaction; for #, $: p<0.05; for *a*, *b*: p < 0.1; data are expressed as mean + SEM.

### EXAMPLES

### MATERIALS AND METHODS

### Cell lines

Human embryonic kidney cells (HEK-293, ATCC CRL-1573), Vero B4 cells and mouse embryonic fibroblasts (MEFs) were maintained in Dulbecco's modified Eagle's medium (DMEM, Gibco) supplemented with 10% FCS and 100 units/ml penicillin and streptomycin, respectively. FKBP51^{-/-} and Akt1/2^{-/-} MEFs (kind gift of Nissim Hay, University of Illinois, Chicago, Illinois) have been described before (Geddes JR and Miklowitz DJ (2013) Lancet 381: 1672-1682; Klengel T et al. (2013) Nat. Neurosci.16: 33-41).

### Primary cultures of murine astrocytes

Enriched astroglial cultures were prepared from postnatal day 1 rat pups (Sprague-Dawley, Charles River, Sulzfeld, Germany) and handled as described by Attwood BK et al. (2011) Nature 473: 372-375 and Hartmann J et al. (2012) Neuropharmacology 62: 332-339.

### FKBP51 Rescue in MEF Fkbip51^{-/-} cells

Afore detached MEF cells (2 x 10⁶) were re-suspended in 100 µl transfection buffer (50 mM HEPES pH 7.3, 90 mM NaCl, 5 mM KCI, 0.15 mM CaCl₂). A maximal amount of 5 µg plasmid DNA was added to the cell suspension, and electroporation was carried out using the Amaxa Nucleofactor system (program # T-020). Cells were re-plated at a density of 10⁵/cm² and further processed for Western blot analysis.

### Plasmids

Plasmids expressing FKBP51-FLAG, HA-Akt1, HA-Beclin1, GFP-Ubiquitin, and myc-Skp2 have been described before (Wochnik GM et al. (2005) J. Biol. Chem. 280: 4609-4616) or were obtained from Addgene.

### Co-Immunoprecipitation (CoIP)

Interaction analysis between Skp2 and the proteins FKBP51, FKBP52, Akt1 and Beclin1 was performed by CoIPs in HEK293 cells. 5×10⁶ cells were electroporated with 5 µg of the respective plasmids expressing tagged versions of the proteins (combination indicated in figure 5) using a GenePulser (Bio-Rad, USA) at 350 V/700 µF in 400 µl of electroporation buffer (50 mM K₂HPO₄/KH₂PO₄, 20 mM KAc, pH 7.35, 25 mM MgSO₄). After three days of cultivation in DMEM/10% FCS, cells were lysed in CoIP-buffer containing 20 mM Tris-HCl pH 8.0, 100 mM NaCl, 1 mM EDTA, 0.5% Igepal complemented with protease inhibitor cocktail (Sigma, P2714). This was followed by incubation on an overhead shaker for 20 min at 4 °C. The lysate was cleared by centrifugation, the protein concentration was determined, and 1.2 mg of lysate was incubated with 2.5 µg myc antibody overnight at 4 °C. 20 µl of BSA-blocked Protein G Dynabeads (Invitrogen, 100-03D) were added to the lysate-antibody mix followed by 3 h incubation at 4 °C. The beads were washed 3 times with PBS and protein-antibody complexes were eluted in 60µl Laemmli buffer for 5 min at 95°C °C. 5-15 µg of the cell lysates or 2.5 µl of the immunoprecipitates were separated by SDS-PAGE.

For the precipitation of Beclin1 (Fig. 4C), HEK cells were transfected with 5 µg GFP-ubiquitin and the immunprecipitation was performed the same way, with the Beclin1 antibody instead of the myc antibody.

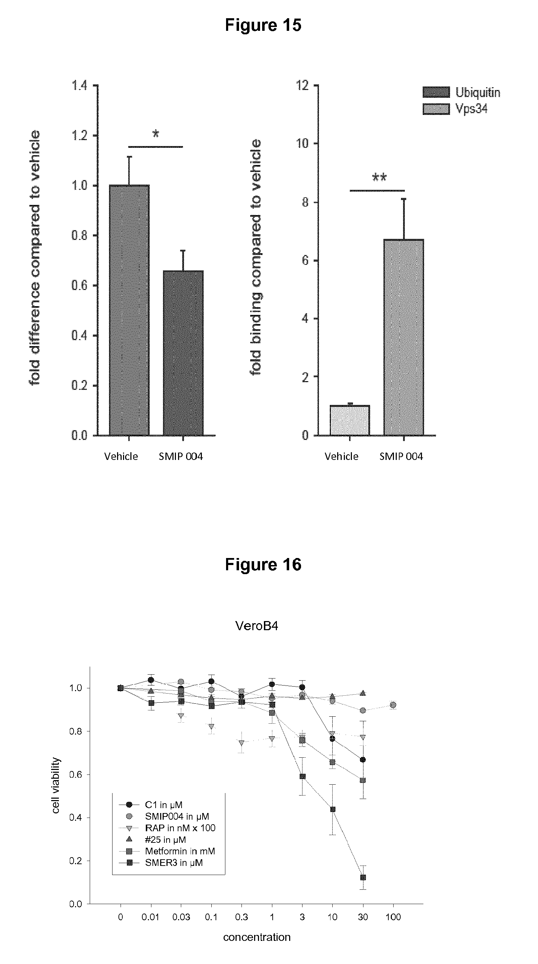

For the precipitation of Beclin1 from brain tissue (Fig. 15), the tissue was first homogenized (300µL of Buffer I (50 mM Tris (pH 7.5), 150 mM NaCl, 5 mM EDTA, Protease inhibitor Mix (Sigma , P2714), Phosphatase Inhibitor (Roche, 04906837001)) followed by sonication and addition of 300µL of Buffer II (50 mM Tris (pH 7.5), 150 mM NaCl, 1% NP40, 1% Deoxycholate, 2% SDS, Protease inhibitor Mix (Sigma , P2714), Phosphatase Inhibitor (Roche, 04906837001)).

### Western Blot analysis

Western blot analysis was performed on eluates from ColPs or with protein extracts from cells or tissue. Protein extracts were obtained by lysing cells or tissue (prelysed as described in the paragraph before) in 62.5 mM Tris, 2% SDS and 10% sucrose, supplemented with protease (Sigma, P2714) and phosphatase (Roche, 04906837001) inhibitor cocktail. Samples were sonicated and heated at 95 °C for 5 min. Proteins were separated by SDS-PAGE and electro-transferred onto nitrocellulose membranes. Blots were placed in Tris-buffered saline, supplemented with 0.05% Tween (Sigma, P2287) and 5% non-fat milk for 1 h at room temperature and then incubated with primary antibody (diluted in TBS/0.05% Tween) overnight at 4 °C. The following primary antibodies were used: Beclin1 (1:1000, Cell Signaling, #3495), Atg12 (1:1000, Cell Signaling #2010), LC3B-I/II (1:1000, Cell Signaling, #2775), FLAG (1:7000, Rockland, 600-401-383), FKBP51 (1:1000, Bethyl, A301-430A), Akt (1:1000, Cell Signaling, #4691), pAkt (1:1000, Ser473, T308, 1:1000, Cell Signaling, #4058, #9275), Actin (1:5000, Santa Cruz Biotechnologies, sc-1616), Skp2, myc (Addgene, 19947), GFP (santa cruz biotechnologies, sc-8334), p27 (cell signaling technologies, 3686), pULK,(S757, cell signaling technologies, 6888) APP-CTF (sigma-aldrich, A8717), DNMT1 (1:1000, Imgenex, IMG-261A), phospho-DNMT1 (S154), (1:1000, Abnova, PAB4924), ptau (S622; Santa Cruz Biotechnologies, #sc-16938).

Subsequently, blots were washed and probed with the respective horseradish peroxidase- or fluorophore-conjugated secondary antibody for 1 h at room temperature. The immuno-reactive bands were visualized either using ECL detection reagent (Millipore, Billerica, MA, USA, WBKL0500) or directly by excitation of the respective fluorophore. Determination of the band intensities were performed with BioRad, ChemiDoc MP, or X-ray-films.

### siRNA

For knock-down of SKP2, the predesign SKP2 siRNA from Abgent was used (#RI14890, Gene Symbol: SKP2-homo-1246).

### Real-Time PCR analysis

Total RNA was isolated from astroglial cells according to the manufacturer's protocol of NucleoSpin RNA II (Macherey-Nagel, Germany). In total, 300 ng of purified RNA was reverse transcribed using random primers (Promega) and Omniscript^{®} Reverse Transcriptase (Qiagen). Based on the LightCycler^{®} System (Roche Applied Science, Germany) and SYBR green detection (QuantiFast^{®} SYBR Green; Qiagen), quantitative assessment of Beclin1 cDNA levels were performed. b-Actin PCR was performed serving as endogenous control for normalization. The primers used were: for Beclin1: antisense - 5'-GACGTTGAGCTGAGTGTCCA-3' sense - 5'-GTCACTGGGGACCTTTTTGA-3'; and for β-Actin: antisense - 5'-CAGGTCCAGACGCAGGATGGC-3', sense-5'-CTACAATGAGCTGCGTGTGGC-3'. Relative changes of gene expression were calculated using the comparative ΔΔC_{T} method (Livak and Schmittgen, METHODS 25, 402-408, 2001). Detection of viral RNA by real-time PCR was as recently described (Corman et al. (2014) Journal of Clinical Virology 60: 168-171).

### Animal treatments and behavioral experiments

The *Fkbp51*^{*-*/*-*} mouse line was previously generated (Touma C et al. (2011) Biol. Psychiatry 70: 928-936) and fully backcrossed to C57/BI6. Male mice (age 10-16 weeks) were injected intraperitoneally with a single dose of either PAR (10 mg kg⁻¹) or saline vehicle. 45 min later, a subgroup of mice (WT vehicle, n=10; Fkbp51^{-/-} vehicle, n=9; WT PAR, n=10; Fkbp51^{-/-} PAR, n = 10; WT) was sacrificed. Hippocampus and prefrontal cortex were extracted. Another subgroup (WT vehicle, n=8; Fkbp51^{-/-} vehicle, n=8; WT PAR, n=9; Fkbp51^{-/-} PAR, n=7) was subjected to a forced swim test (6 min test duration) 45 min after the injection.

### Acute amitriptyline (AMI) treatment:

Male mice (age 10-16 weeks) were injected intraperitoneally with a single dose of either AMI (10 mg kg⁻¹) or saline vehicle. 45 min later, a subgroup of mice (WT vehicle, n=10; 51 KO vehicle, n=9; WT AMI, n=9; 51 KO PAR, n=11) were subjected to a forced swim test. Another subgroup (WT n=5, KO n=5) was sacrificed, and brain tissue and blood was sampled for analysis of drug concentrations.

### Chronic PAR treatment:

### Design of the chronic stress and chronic PAR treatment experiment

We analysed the ability of 51KOmice to recover from CSDS and the effects of chronic PAR treatment during a 3-week recovery period. Initially, wild-type and 51 KOmice were split into 2 x 2 groups (control WT, control FKBP51^{-/-}, defeat WT, defeat FKBP51^{-/-}) and subjected to the chronic stress procedure described below. After cessation of the stressor, groups were subdivided into vehicle-treated and paroxetine-treated animals, resulting in 8 groups (n = 7-13 per group). The treatment phase lasted for three weeks. The behavioral tests took place during the last week of the paroxetine treatment.

### Chronic Stress Procedure and Physiological Parameters

The CSDS paradigm lasted for 21 days. Briefly, the experimental mice were introduced into the home cage (45 cm x 25 cm) of a dominant resident mouse and defeated shortly after. When the defeat was achieved, the animals were separated by a wire mesh, preventing physical but allowing sensory contact for 24 h. Each day, stressed animals were defeated by another unfamiliar, dominant resident mouse in order to exclude a repeated encounter throughout the experiment. The daily defeat was performed between 11:00 and 16:00 h; varying starting times reduced the predictability of the stressor and therefore minimized a potential habituation effect. Experimental mice were always defeated by resident males during the entire stress period. Control mice were housed in their home cages during the course of experiment. Both stress and control animals were handled daily during the stress procedure; body weight for all mice was assessed at the beginning of the experiment, after the cessation of the stress period, and on the day of killing. Animals that underwent the stress procedure were subsequently single housed in standard cages.

### Chronic PAR treatment

Paroxetine (GlaxoSmithKline, Munich, Germany) was administered via drinking water. Briefly, the paroxetine solution was diluted in tap water to a final concentration of 0.16 mg/ml. With average water consumption of 5ml/mouse/day, the daily dose of paroxetine was ~20 mg/kg body weight. Fluid intake was monitored daily and the variation of fluid intake was found to be <10% over the course of experiment.

### Behavioral Analyses

The behavioral tests were carried out between 08:30 and 12:30 h in the same room in which the mice were housed. The testing order was as follows: Open-field (OF), social avoidance (SA), forced swim test (FST), and acute stress response. All tests were analyzed using an automated videotracking system (Anymaze 4.20, Stoelting, Wood Dale, IL). All animals underwent the same testing battery in the same order of tests. To minimize possible carryover effects of the different behavioral tests, the sequence of tests was arranged from the least stressful to the most stressful, with a minimum of 24 h between tests.

### Open-field test

The open-field test was performed to investigate locomotion differences. Testing was carried out in an empty open-field arena (50 cm x 50 cm x 50 cm) made of gray polyvinyl chloride (PVC), which was evenly illuminated with 15 Lux. The low illumination of the open field arena was chosen to specifically investigate locomotion behavior and not create an aversive center region that may induce anxiety-related behavior. Testing time was 10 min and the main parameter of interest was the total distance travelled.

### Social avoidance test

Briefly, animals were allowed to explore the open field arena for 2.5 min with an empty wire mesh cage placed at one side of the apparatus. In a second stage, the animals were confronted with an unfamiliar CD1 resident mouse in the wire mesh cage for another 2.5 min. The ratio between the time in the interaction zone of the no-target trial and the time in the interaction zone of the target trial serves as a marker for disturbed social behavior associated with depressive disorders. Animals that did not explore the interaction zone at all were excluded from the analysis (wt ctrl veh = 1; 51 KOctrl veh = 1; wt ctrl PAR = 2; 51 KOctrl PAR = 1; wt stress veh = 1; 51 KOstress veh = 1; wt stress PAR = 1).

### Forced swim test

In the forced swim test, each mouse was put into the a 2 l glass beaker (diameter: 13 cm, height: 24 cm) filled with tap water (21 ± 1 °C) to a height of 15 cm, so that the mouse could not touch the bottom with its hind paws or tail. Testing duration was 5 min. Time spent immobile (floating) and time spent struggling was scored by an experienced observer, blind to treatment or condition of the animals.

### Acute stress response

The FST also served as an acute stressor in order to determine the stress response by measuring corticosterone plasma concentrations. After the FST, all mice were towel-dried and placed into their home cage to recover from the acute stressor. Blood samples were taken by tail cut. 30 min (stress response) and 90 min (stress recovery) after the onset of the FST. Samples were collected in 1.5 ml EDTA-coated microcentrifuge tubes (Kabe Labortechnik, Germany). All blood samples were kept on ice and later centrifuged at 8000 rpm at 4 °C for 15 min. Plasma was transferred to new, labeled tubes and stored at -20 °C until determination of corticosterone by radioimmunoassay (MP Biomedicals Inc; sensitivity 12.5 ng/ml).

### Sampling Procedure

24 hours after the last defeat, blood samples were taken by tail cut as described above, in order to assess the plasma corticosterone levels immediately after the chronic stress paradigm. 3 weeks after the last defeat, thus following 3 weeks of treatment, all animals were killed by decapitation following quick anesthesia by isoflurane at the end of the experiment. Basal trunk blood samples were processed as described above. Adrenal and thymus glands were removed, dissected from fat, and weighed. Prefrontal cortex and hippocampus were prepared for protein analysis.

### Statistical Analysis

The data presented are shown as means +SEM, analyzed by the commercially available software SPSS 16.0. When two groups were compared, the student's t-test was applied. For four or more group comparisons, 2-way or 3-way analysis of variance (ANOVA) was performed. If interaction effects (p < 0.05) were observed Tukey post-hoc tests were applied. A nominal level of significance p < 0.05 was accepted.

### Electrolphysiology

Brain slices were obtained from 11-18 week-old male FKBP51+/+ and FKBP51-/- litter mates (Fig. 1) or from BL6 mice (Fig. 7). Preparation and staining of slices with the voltage-sensitive dye Di-4-ANEPPS as well as VSDI and data analysis were performed essentially as described (von Wolff et al. (2011) J. Psychiatr. Res. 45: 256-261; Refojo et al. (2011) Science 333: 1903-1907). Four acquisitions subsequently recorded at intervals of 5 s were averaged. Neuronal activity was evoked by square pulse electrical stimuli (200 µs pulse width) delivered via a custom-made monopolar tungsten electrode to the Schaffer collateral-commissural pathway. The circular ROI was placed into the CA1 stratum radiatum near the stimulation electrode. The ROI also covered a part of the adjacent CA1 stratum pyramidale (Fig. 1 B). The intensity of voltage stimulation was adjusted to produce FDSs with peak amplitudes of ~30% of the highest attainable value.

### Aβ42 ELISA

Aβ42 was determined in brain lysates using a commercially available kit according to the manufacturer's instructions (Life Technologies, KMB3441)

### Viral infections

Viral infections were as recently described(Corman et al. (2014) Journal of Clinical Virology 60: 168-171). Briefly, VeroB4 cells were diluted to a concentration of 34 x 10⁵/ml and 500µl were placed per well of a 24 well plate. The next day, cells were infected with Middle East respiratory syndrome coronavirus (MERS-CoV) at MOI of 0.0001 for 1 h at 37°C. Cells were washed two times with PBS and incubated in DMEM supplemented with drug or vehicle as indicated. Probes were taken 24h, 48h and 72h after infection. Pretreatment and cotreatment with compound was as described in the figures.

### Ethics statement

The animal experiments were carried out in accordance with the European Communities' Council Directive 2010/63/EU. All efforts were made to minimize animal suffering during the experiments; all experiments were based on the 3R principles: reduction, refinement, replacement. The protocols were approved by the committee for the Care and Use of Laboratory animals of the Government of Upper Bavaria, Germany (license number 55.2-1-54-2532-48-11).

Approval for the MARS project was received by the ethics committee in charge (submission number 318/00, ethics committee of the Medical Faculty at the Ludwig Maximilians University Munich, Germany), and participants gave oral and written consent before study inclusion.

### Preparation of human PBMCs

Blood was collected via venipuncture, diluted with PBS and carefully loaded on Biocoll solution (BioChrom AG, L6113) and centrifuged at 800 x g for 20 min (brakeless running down). PBMCs were enriched by selecting the interphase of the Biocoll gradient and washed two times with ice-cold PBS. PBMCs were re-suspended in RPMI and plated at 4x105/cm². After recovery for 6 h, cells were treated with either 190 µM (8 µg ml⁻¹) lithium chloride, or 694 µM (100 ng ml⁻¹) VPA, or 365 nM (120 ng ml⁻¹) PAR. Concentrations had been chosen to match therapeutic concentrations in the serum according to the consensus guidelines for therapeutic drug monitoring in psychiatry.

### Human samples

Healthy subjects: Protein-protein correlations (Fig. 2A-C, 10) were determined in extracts from PBMCs from 21 healthy male subjects (25.8±2.7 yrs). FKBP51-dependent antidepressant effects (Fig. 2D-F, 10) were evaluated in cultivated PBMCs from another 20 healthy male subjects (age 34.8±6.9 yrs).

Patients: 51 consecutive participants (26 females, 25 males; age: 49.0 ± 14.7 yrs) of the Munich Antidepressant Response Signature (MARS) project were included for protein expression analyses and PBMC cultivation and *ex vivo* treatment with antidepressants (Figures 3, 11, 23). MARS is an open-label prospective clinical study conducted at the hospital of the Max Planck Institute of Psychiatry Munich and collaborating hospitals in South Bavaria and Switzerland to evaluate typical depression courses under standard antidepressant treatment (Hennings JM et al. 2009 Clinical characteristics and treatment outcome in a representative sample of depressed inpatients - findings from the Munich Antidepressant Response Signature (MARS) project. J. Psychiatr. Res. 43: 215-229). Patients were diagnosed according to DSM-IV criteria (American Psychiatric Association (1994) Diagnostic and statistical manual of mental disorders: DSM-IV-TR. 4th ed. American Psychiatric Association, Washington DC) Patients suffered from major depression, recurrent depression or from a depressive episode within a bipolar disorder (n=6). Depression severity was assessed by using the 21-item Hamilton Depression rating scale (HDRS) (Overall JE and Rhoades HM (1982) Use of the Hamilton Rating Scale for classification of depressive disorders. Compr. Psychiatry 23: 370-376) with an average baseline score of 23.8 (SD 5.6), indicating severe depression. Patients received antidepressant treatment in a naturalistic study setting (Hennings JM et al. 2009 Clinical characteristics and treatment outcome in a representative sample of depressed inpatients - findings from the Munich Antidepressant Response Signature (MARS) project. J. Psychiatr. Res. 43: 215-229). Antidepressant treatment outcome was evaluated as percent change in the HDRS scores between baseline and after six weeks of treatment. Fasting venous blood samples for extraction of PBMCs were drawn from patients between 8 and 9 am within 5 days after admission.

Another set of probes from 55 patients was analyzed by antibody array (Zeptosens, Bioanalytical Solutions, Switzerland) (Fig. 11A,B).

### Statistical analysis

When two groups were compared, the student's t-test was applied. For three or more group comparisons, one-way, two-way or three-way analysis of variance (ANOVA) was performed, followed by Tukey's post-hoc test, as appropriate.

Protein-protein associations were analyzed using Pearson correlations. In the clinical sample, associations between human protein expression and antidepressant treatment outcome as well as protein-protein interactions were evaluated with Pearson correlations after correction for the effects of age and gender (partial correlation). p<0.05 was considered significant.

### Example 1: FKBP51 dependent effects of paroxetine on animal behavior and on synaptic function.

To assess whether FKBP51 modulates AD response in mice, *Fkbp51*^{+/+} and *Fkbp51*^{-/-} mice were treated with the antidepressant paroxetine (PAR). A well-established test for AD action was employed: *Fkbp51*^{-/-} -mice were subjected to the forced swim test to monitor the animals' response to an acute PAR injection (10 mgkg⁻¹). PAR-treated *Fkbp51*^{+/+} mice spent less time floating (immobility) compared to vehicle-treated *Fkbp51*^{+/+} mice, thus exhibiting the expected response to ADs. In contrast, the effect of PAR was significantly less pronounced in *Fkbp51*^{-/-} mice (Fig. 1A). Similarly, PAR treatment led to an increase in the time spent struggling in *Fkbp51*^{+/+} mice. This effect of PAR was significantly attenuated in *Fkbp51*^{-/-} mice (Fig. 1A). The inventors performed an additional forced swim test administering the antidepressant amitriptyline in FKBP51 WT and FKBP51 KO mice (Fig. 1 B). Similar to paroxetine, the behavioral effects as well as effects on autophagy markers mediated by amitriptyline were only observed in FKBP51 WT mice but not in FKBP51 KO mice. These data point to a more general role of FKBP51 in the action of antidepressants.

To evaluate molecular effects of ADs that may depend on FKBP51 and parallel the behavioral phenotype, autophagy markers were analyzed in protein extracts from the hippocampus and prefrontal cortex after PAR injection (10 mgkg⁻¹). In *Fkbp51*^{+/+} mice, PAR treatment increased the levels of Beclin1, Atg12, and Vps34, increased the ratio of LC3B-II/I, and evoked dephosphorylation of the kinase Akt (phosphorylation at S473 in Akt1 represents the activated form, subsequently referred to as "pAktS473"). In contrast, *Fkbp51*^{-/-} mice showed no alterations in protein levels of autophagy markers and pAktS473 in either brain region after PAR treatment (Fig. 1D, E). The results in *FKBP51*^{*-*/*-*} mice suggest a role of FKBP51 in autophagic events, which was further evaluated using biochemical and cell biological methods.

Irrespective of the question whether AD-induced increase of autophagic markers in all cases indicates enhanced protein turnover, autophagic pathways have been linked to synaptic function. Since the latter is more obviously linked to behavior and diseases such as depression, it was tested whether FKBP51 changes the effect of PAR on neuronal function. Hippocampal CA3-CA1 synapses were electrically stimulated in brain slices from *Fkbp51*^{+/+} and *Fkbp51*^{-/-} mice, and CA1 neuronal activity was monitored by means of voltagesensitive dye imaging (VSDI). This approach was chosen for two reasons. First, the CA1 area is an important output subfield of the hippocampus, which has been implicated in the pathophysiology of depression and in AD action. Second, considering potential heterogeneity of the PAR effect at CA3-CA1 synapses, VSDI measurement of neuronal activity in a larger portion of CA1 appeared more relevant than the typically more restricted electrode recording techniques. In slices from Fkbp51^{+/+}-mice, bath application with PAR (10 µM) enhanced the 'region of interest' (ROI)-extracted fast, depolarization-mediated imaging signals (FDSs), which reflect excitatory postsynaptic potentials as well as neuronal action potentials. This effect was not observed in slices from *Fkbp51*^{-/-}animals (Fig. 1F and C).

### Example 2: The effects of chronic PAR treatment on behavior and autophagic pathways in mice depend on FKBP51.

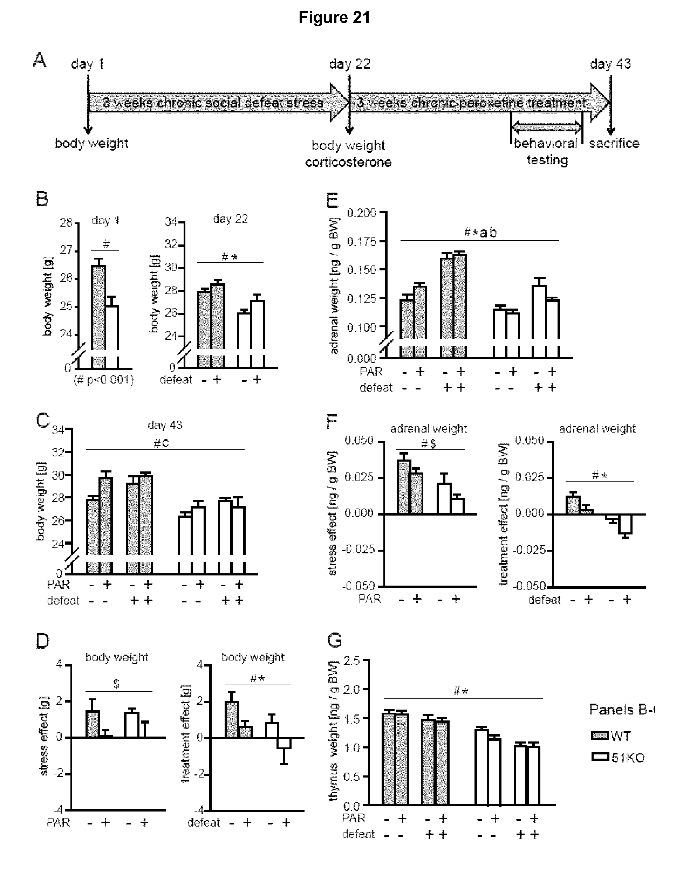

To more closely mimic the clinical situation, the effect of FKBP51 deletion in mice after chronic PAR treatment was also assessed; in addition, chronic social defeat stress (CSDS) as it is suggested to model some endophenotypes of depression was applied. Mice were subjected to three weeks of CSDS, followed by three weeks of PAR treatment and behavioral tests during the last week (Fig. 21A).

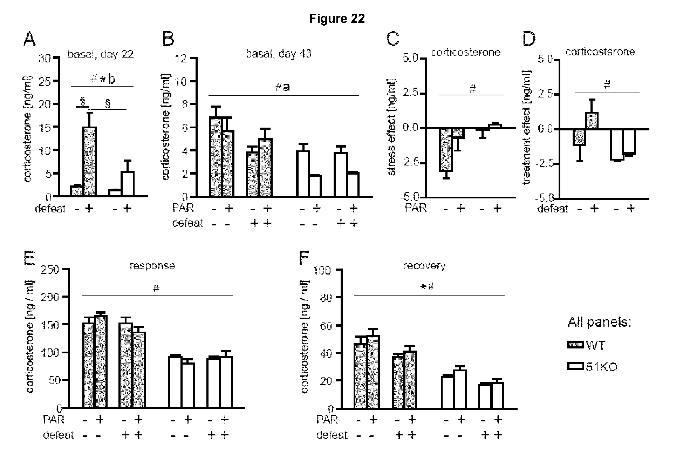

The results determined from body weight, adrenal weight, thymus weight and corticosterone levels show that FKBP51 shapes the long lasting physiological and neuroendocrine effects of CSDS (Figs. 21 and 22).

On the day of sacrifice (day 43, after 3 weeks treatment), we found a main genotype effect and a condition x treatment interaction for body weight (Fig. 21 C) could be found. In order to isolate genotype-dependent stress and treatment effects, the inventors proceeded to normalize the data to either non-stressed controls or vehicle treatment. First, all stressed groups were normalized by assessing the difference in body weight compared to their corresponding unstressed control of the same genotype and treatment. The inventors found a significant treatment effect, because stress-induced increase of body weight was significantly more pronounced in vehicle treated mice than in paroxetine treated mice (Fig. 21D left). To isolate stress effects as well as genotype-dependent treatment effects, lized all paroxetine treated groups were normalized by assessing the difference in body weight compared to their corresponding vehicle treated control of the same genotype and condition. 2-way ANOVA revealed a significant condition and genotype effect. Paroxetine-induced body weight increase was significantly enhanced in mice under basal conditions compared to stressed animals. Interestingly, 51 KO mice were significantly less affected by paroxetine-induced body weight increase compared to wild-type mice (Fig. S21 D right).

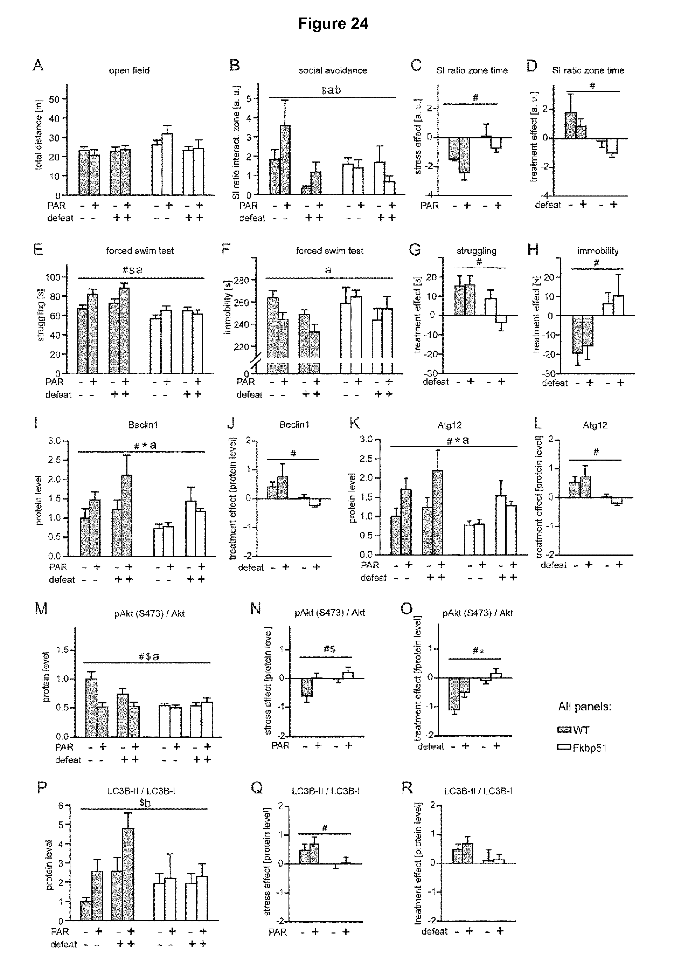

To assess the behavioral effects of FKBP51 deletion on the actions of CSDS and chronic PAR treatment a social interaction paradigm was used in addition to the forced swim test. Moreover analysis of general locomotion in the open field to control for potential confounding effects was performed. The total distance traveled did not differ between genotype, condition or treatment groups (Fig. 24A). In the social avoidance test, deletion of FKBP51 resulted in loss of effects by CSDS and by chronic PAR treatment. 3-Way ANOVA revealed a main treatment effect, a genotype x condition interaction and a genotype x treatment interaction (Figure 24B). To isolate treatment effects and genotype-dependent stress effects, all stressed groups were normalized by assessing the difference in the social interaction ratio compared to their corresponding unstressed control of the same genotype and treatment. It was found that CSDS significantly reduced social behavior in wild-type, but not in 51 KO mice (Figure 24C). To isolate stress effects and genotype-dependent treatment effects, all paroxetine treated groups were normalized by assessing the difference in the social interaction ratio compared to their corresponding vehicle treated control of the same genotype and condition. Paroxetine significantly enhanced social behavior in wild-type, but not in 51 KO mice, both in CSDS-exposed and control animals (Figure 24D). In the forced swim test, chronic PAR treatment was significantly less effective in enhancing struggling and reducing floating in FKBP51-deleted mice compared to wild type mice. A genotype x treatment interaction for the parameters struggling and floating (Figure 24E and F) was observed. To isolate stress effects and genotype-dependent treatment effects, the inventors normalized all paroxetine treated groups by assessing the difference in struggling and floating compared to their corresponding vehicle treated control of the same genotype and condition. In both parameters, 51 KO mice were significantly less affected by paroxetine treatment compared to wild-type mice, independent of condition (Figure 24G and H). The parameter swimming did not differ between genotype, condition or treatment groups (data not shown).

These data support the conclusion that FKBP51 also impacts on the effects of chronic PAR treatment.

### Example 3: Effects of ADs in blood cells of healthy individuals depend on FKBP51

In mice lacking FKBP51 (example 1) it was found that effects of the antidepressant paroxetine on behaviour and on synaptic function were abolished. These findings could be translated to humans, by analysing peripheral blood mononuclear cells (PBMCs) from healthy individuals and also from inpatients suffering from depression.

PBMCs derived from healthy male subjects showed marked variations in basal FKBP51 expression (Figs. 2A-C). The FKBP51 expression level was positively correlated with the expression of Beclin1, and negatively correlated with the phosphorylation status of Akt (S473); LC3B-II/I displayed a trend correlation with FKBP51 protein levels.

PBMCs from healthy male subjects were also cultivated ex vivo and treated with PAR for 48 h. The extent of de-phosphorylation of pAkt^{S473}, change of LC3B-II/I ratio, and levels of Beclin1 in response to ADs correlated with the expression level of FKBP51 (Figs. 2D-F). Higher expression of FKBP51 was associated with AD induced de-phosphorylation of pAkt^{S473}, and lower expression of FKBP51 was associated with AD-enhanced pAktS473. With increasing FKBP51 levels, a gradual shift from slightly inhibitory to stimulatory effects of ADs on Beclin1 expression was observed. The AD triggered lipidation of LC3B-I was positively correlated with the FKBP51 expression status.

### Example 4: Clinical improvement of depressive patients is predicted by their levels of Beclin1, pAkt and FKBP51 at hospital admission and by the effects of antidepressants in their ex vivo cultivated blood cells.

Further evaluation of the clinical relevance of the above findings the expression levels of Beclin1, FKBP51, and pAkt^{S473}/Akt in PBMCs from inpatients (M.A.R.S. - Munich antidepressant response signature) with a current depressive episode at admission were determined and compared with the clinical treatment response. The clinical response after 6 weeks of treatment showed statistically significant positive correlations with the levels of Beclin1 and FKBP51, and a negative correlation with pAkt^{S473}/Akt (Figs. 3A-C).

Furthermore, it could be demonstrated that the response of these cells in culture to antidepressants, determined by the expression of Beclin1, pAkt and LC3B-II/I could be predictive for the success of antidepressants in the clinic (Figure 3D-I).

To this end, PBMCs were collected from inpatients at admission, cultivated and treated with amitriptyline (AMI), fluoxetine (FLX), or PAR ex vivo. A correlation between therapeutic outcome and the increase of Beclin1 levels in treated PBMCs for all three ADs were observed (Figs. 3D-F). There was a significant negative correlation between therapeutic outcome and the change of pAktS473 upon treatment with AMI or PAR (Fig. 3G,H). There was a significant correlation between therapeutic outcome and induction of LC3B-II/I in the case of treatment with PAR (Fig. 3I), but not with AMI or FLX (not shown). Thus, the levels of FKBP51, pAkt^{S473}/Akt and Beclin1 at admission, as well as the response to AD treatment in PBMCs, correlate with therapeutic outcome.

### Example 5: pDnmt1 as state marker and as predictor

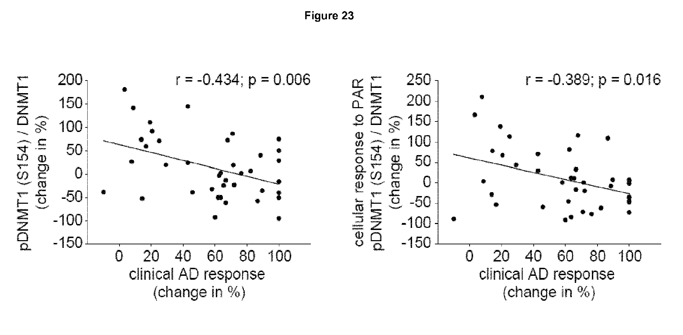

The levels of Dnmt1 and of phosphorylated Dnmt1 (serine 154) was determined in PBMCs from depressive inpatients at admission and 6 weeks after treatment in the clinic (see Fig. 23 left panel). The change in the levels of phosphorylated Dnmt1 (normalized to total Dnmt1) from admission to 6 weeks later was correlated to the response to clinical treatment expressed as percent change in the score of the 21-items Hamilton Depression Rating Scale between admission and after 6 weeks of treatment.

PBMCs from depressive inpatients were obtained at admission and treated with paroxetine or vehicle for 48 h (see Fig. 23 right panel). The change in the levels of phosphorylated Dnmt1 (serine 154, normalized to total Dnmt1), i.e. the cellular response to antidepressant treatment, was correlated to the response to clinical treatment expressed as percent change in the score of the 21-items Hamilton Depression Rating Scale between admission and after 6 weeks of treatment.

### Example 6: Determination of involvement of Skp2 in mechanisms of Beclin1 stabilization

The results above raised the question whether induction of autophagic pathways by a small molecule inhibitor leads to antidepressant-like effects in neurotransmission and on behavior.

Rat cortical astrocytes were transfected with expression vectors encoding for FKBP51 or FKBP52, or were stimulated with amitriptyline (AMI, 10µM), fluoxetine (FLX, 10µM) or paroxetine (PAR, 10µM) for 72h. Beclin1 mRNA expression levels were determined by qPCR. HEK cells were treated either with proteasome inhibitor MG132 or with vehicle for 5h and Beclin1 protein levels were determined by Western analysis. Furthermore Co-immunoprecipitations were performed with Beclin1 antibody in HEK cell lysates with ectopic Ub-GFP.

HEK cells were transfected with plasmids encoding FLAG-tagged FKBP51/52, HA-tagged Beclin1 or Akt1 and myc-tagged Skp2 constructs. After precipitation of protein complexes using a myc-antibody and (co)precipitated proteins were quantified by Western blots.

*In vivo* and *in vitro* experiments showed that antidepressant treatment elevates protein levels of the autophagic initiator Beclin1 and further triggers autophagic signaling. The inventors found that Beclin1 protein level as well as its activity status is regulated by posttranslational modifications such as phosphorylation and ubiquitination (Figure 4) through formation of a heterocomplex with the protein kinase Akt and the E3 ligase Skp2, which is scaffolded by FKBP51 (Figure 5). Active Akt phosphorylates and thereby activates Skp2, resulting in ubiquitination and subsequent proteasomal degradation of Beclin1 (Figure 4/5). In the presence of FKBP51, the protein phosphatase PHLPP is recruited to Akt, de-phosphorylation inactivates both the kinase and the E3 ligase, thereby protecting Beclin1 from UPS (ubiquitin proteasome system) mediated degradation. Knock-down of Skp2 by siRNA demonstrated a strong stabilizing effect on Beclin1 protein level (Figure 5 C).

### Example 7: Identification of a small molecule inhibitor of Skp2 with low cell toxicity and anti-depressant effects

In an effort to find a small molecule to inhibit the E3 ligase Skp2 three promising candidates of published chemical genetic screens were tested on cellular toxicity and its potency to stabilise Beclin1. Cytotoxicity was determined by an MTT assay performed in HEK 293 cell and in primary rat cortical astrocytes. Effects of small compounds on Beclin1 and p27 protein levels were determined by Western analysis of HEK 293 primary rat cortical astrocytic lysates (see Fig. 6). N-(4-butyl-2-methylphenyl)acetamide (SMIP004) was identified as a potent stabilizer of Beclin1 with very low cytotoxic effects in primary astrocytes. It is noteworthy that the effect of SMIP004 is particularly strong in primary astrocytes (Fig. 6E).

These findings were confirmed in various cell types as well as in mice brain lysates of animals treated with SMIP004 (Figures 6 and 8D, E).

Furthermore the neuronal activity in CA1 hippocampus was measured after treatment with SMIP004 (Figure 7). Therefore C57BL/6 mice were treated with SMIP004 (0.5 mgkg-1 or 5 mgkg-1), par-oxetine (10 mgkg-1) or vehicle and subjected to the open field test (Fig. 8A-E) and the tail suspension test (F; n=10 per group). Mice were sacrificed after behavioral testing. And protein levels were determined in extracts of the hippocampus and the prefrontal cortex.

Inhibition of Skp2 using SMIP004 increased synaptic neurotransmission similarly to paroxetine (Figures 7, 1C). In the tail suspension test, a behavioral paradigm to determine antidepressant-like effects in rodents, SMIP004 treated C57BL/6 mice revealed an antidepressant-like behavior comparable to animals treated with paroxetine (Figure 8). Similar effects were observed with Balb c mice (not shown). All these data substantiate the concept that pharmacological targeting of FKBP51-directed pathways as exemplified with the Skp2 inhibitor has the potential to elicit antidepressant-like effects (Figure 9).

### Example 8: GSK3β kinase activity in PBMCs of depressive patients and the cellular response to psychoactive drugs

To translate the cellular and animal findings to humans, first the expression levels of FKBP51, pGsk3β^{S9}/Gsk3β and β-catenin were determined in peripheral blood mononuclear lymphocytes (PBMCs) of healthy volunteers. A highly significant correlation between FKBP51 expression and pGsk3β^{S9}/Gsk3β was observed (Fig. 10A), while beta-catenin showed only a trend correlation with FKBP51 (not shown). To evaluate the relationship between the effects of psychoactive drugs on pGsk3β^{S9}/Gsk3β and the FKBP51 expression status, PBMCs were cultivated *ex vivo* and treated with lithium, PAR or VPA. To more closely mimic the clinically relevant *in vivo* conditions, drug concentrations were chosen based on standard values of therapeutic drug monitoring in psychiatry. The effect of lithium and PAR on pGsk3β^{S9}/Gsk3β showed a significant positive correlation with the expression level of FKBP51 (Fig. 10B, C), while the effect of VPA was independent of the FKBP51 expression status (not shown).

Since evidence has been provided that stress can lead to enhanced pGsk3β^{S9}/Gsk3β under certain circumstances, we hypothesized that this might be linked to the induction of FKBP51. We determined the levels of pGsk3β^{S9}/Gsk3β and FKBP51 in PBMCs from healthy male subjects before and 6 h after administration of the synthetic glucocorticoid dexamethasone (DEX, 1.5 mg). There was a significant correlation between the induction of FKBP51 and the increase in pGsk3β^{S9}/Gsk3β (Fig 10D).

### Markers of GSK3β activity in PBMCs of depressive patients and the cellular response to psychoactive drugs correlates with clinical improvement

To further test the physiological relevance of the FKBP51-dependent actions of psychoactive drugs on GSK3β and downstream targets, we measured protein levels in PBMCs from 55 patients using a protein array and related them to clinical treatment response. We observed a significant negative correlation of the GSK3β target pTau (S622) with clinical treatment response (Fig. 11A, B); a trend was received for pGSK3β^{S9}/GSK3β. In another group of 43 patients, we applied conventional Western blotting and found a significant correlation of pGsk3β^{S9}/Gsk3β at the time of admission to the hospital and clinical improvement 6 weeks later (Fig. 11 C). Moreover, we cultivated PBMCs from patients at the time of admission to the hospital and treated them with psychoactive drugs. The cellular response of pGsk3β^{S9}/Gsk3β upon exposure to lithium or PAR significantly correlated with clinical improvement (Fig. 11D, E), while the correlation was not significant in the case of VPA (not shown).

### Example 9: Investigation of N-(4-butyl-2-methylphenyl)acetamide for the treatment of Alzheimer's disease

### Experimental Set-up:

TG2576 animals (used as Alzheimer's disease model, overexpress a mutant form of amyloid precursor protein (APP)) were chronically treated with daily intraperitoneal injections of SMIP004 (*N*-(4-butyl-2-methylphenyl)acetamide; 5mg/kg) or vehicle (0.9% NaCl; 1.25% DMSO) for 3 months. Brain and other tissue was analyzed for markers of autophagy and for the levels of soluble beta-amyloid (Aβ). It is commonly assumed that Aβ agglomerates are causally linked to Alzheimer's disease.

### Results:

The major results are that:
1. SMIP004 reduces the levels of soluble Aβ in plasma and CSF (Fig. 12)
2. SMIP004 reduces the levels of APP-CTF in the brain (Fig. 13)
3. SMIP004 induces autophagy, independently of the mTOR-ULK1 cascade (Figs 14, 15)

These results indicate that SMIP004 not only elicits autophagic pathways, but also could have a beneficial effect in treating Alzheimer's disease, evidenced by the reduction in the precursor protein APP and the reduction in the levels of soluble Aβ. Gene mutations causing familial forms of Alzheimer's disease have been found to increase Aß levels.

### Example 10: Investigation of antiviral activity of N-(4-butyl-2-methylphenyl)acetamide

### Background:

Some viruses use double membrane vesicles for cell entry. These vesicles are spiked with autophagosomal LC3. In the case of corona viruses, vesicles induced by these viruses are not degraded, which leads to the cellular incorporation of the corona viruses. Thus, the idea was to increase functional autophagy to degrade CoV-positive vesicles.

### Experimental set-up:

Several reported Skp2 inhibitors were chosen, as well as known autophagy inducers. The experiment had three parts: 1. Determining the substance tolerance in cells. 2. Preincubation of the cells with the substances prior to virus infection (can the substances prevent infection?). 3. Incubation of the cells with the substances after virus infection (are the substances curative).

### Results:

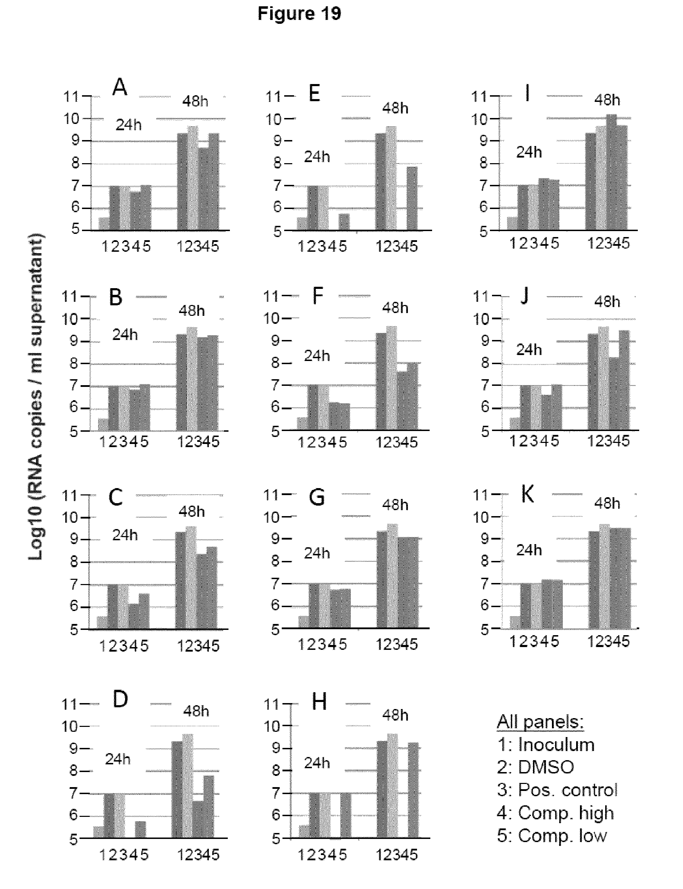

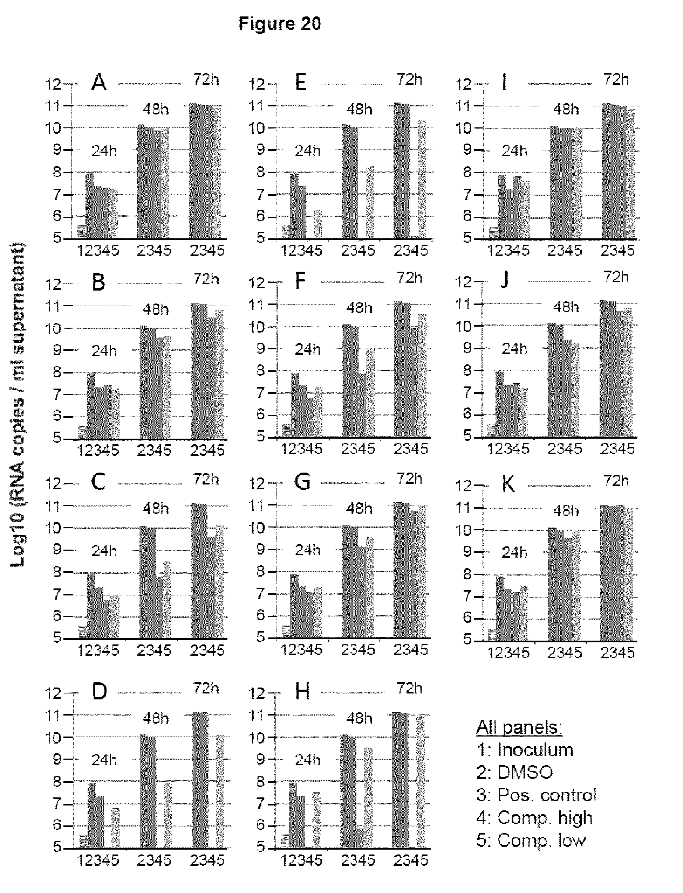

1. Skp2 inhibitors vary in their cell toxicity (Fig. 16). Not all Skp2 inhibitors induce autophagy (Fig. 17).
2. Only Skp2 inhibitors that induce autophagy (stabilize beclin1 and induce LC3 lipidation) protect cells from cell death after viral infection (pretreatment, Figs 18 & 19).
3. Skp2 inhibitors also protect cells when applied at the time of viral infection (Fig. 20).

All data presented here were obtained with the MERS virus.

These data suggest the potential of Skp2 inhibitors, and of SMIP004 and derivatives in particular, in the treatment of viruses, and of corona viruses in particular. This might be of special interest in the case of the MERS virus, which currently is on the verge of spreading world-wide, possibly endemic, and for which no efficient treatment exists to date.

## Claims

1. A compound of the general formula I: 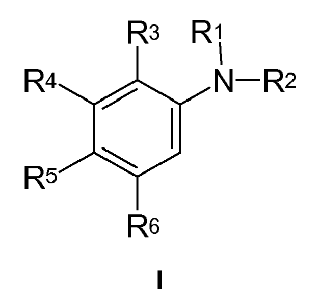 wherein
R¹ and R² are independently of each other selected from -R⁷, -R⁸, -COR⁷, -COR⁸, -CO₂R⁷, -CO₂R⁸, -(CR¹⁰R¹¹)ₙR¹², -(CR¹³R¹⁴)ₘ-R¹⁵, -(CR¹⁰R¹¹)ₙ-O-R⁷, -(CR¹³R¹⁴)ₘ-OR⁸, -(CR¹⁰R¹¹)ₙ-SR⁷, -(CR¹³R¹⁴)ₙ-SR⁸, -(CR¹⁰R¹¹)ₙ-NR⁹R⁷, -(CR¹³R¹⁴)-NR¹⁶R⁸, -CR¹⁰R¹¹-O-CR¹²R¹³R¹⁵, -CR¹⁰R¹¹-S-CR¹²R¹³R¹⁵, -CR¹⁰R¹¹-NR⁹-CR¹²R¹³R¹⁵, -CR¹⁰R¹¹-CR¹²R¹³-R⁷;
and R¹ and R² can form together with the nitrogen atom they are connected to a heterocycle selected from: 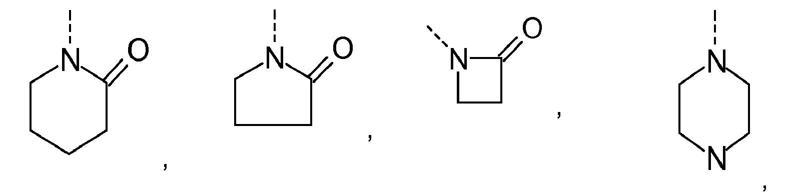 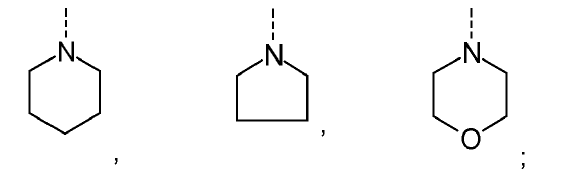 R³ is selected from: -H, -F, -Cl, -Br, -I, -CH₃, -C₂H₅, -C₃H₇, -C₄H₉, -C₅H₁₁, -C₆H₁₃;
R⁵ is selected from: -CH₃, -C₂H₅, -C₃H₇, -C₄H₉, -C₅H₁₁, -C₆H₁₃, -C₇H₁₅, -C₈H₁₇, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -C≡CH, -C≡C-CH₃, -CH₂-C≡CH, -C₂H₄-C≡CH, -CH₂-C≡C-CH₃, -C≡C-C₂H₅, -C≡C-C₃H₇, -CH₂-C≡C-C₂H₅, -C₂H₄-C≡C-CH₃, -C₃H₇-C≡CH, 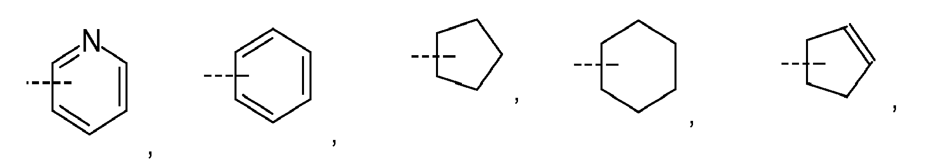 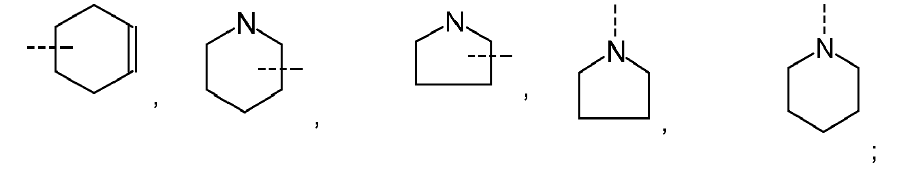 R⁴ represents H or R⁴ and R⁵ form together with the two aromatic carbon atoms they are connected to a cycle selected from: 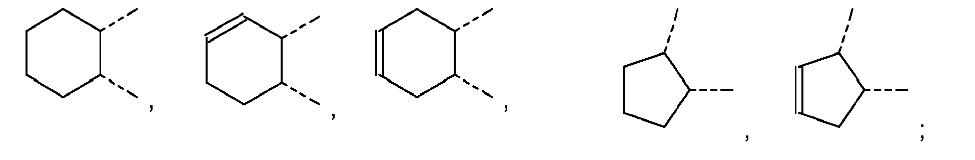 R⁶ represents H or R⁶ and R⁵ form together with the two aromatic carbon atoms they are connected to a cycle selected from: 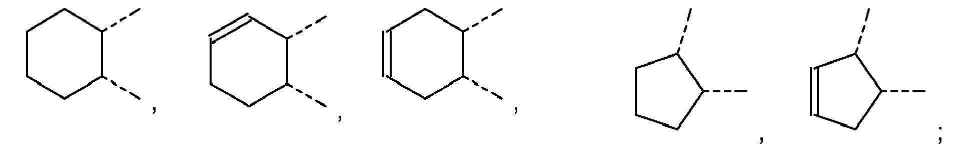 R⁷ and R⁸ are independently of each other selected from: -H, -CH₃, -C₂H₅, -C₃H₇, -C₄H₉, -C₅H₁₁, -C₆H₁₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -CH₂-C≡CH, -C₂H₄-C≡CH, -CH₂-C≡C-CH₃, -CH₂-C≡C-C₂H₅, -C₂H₄-C≡C-CH₃, -C₃H₇-C≡CH;
R¹⁰, R¹¹, R¹², R¹³, R¹⁴ and R¹⁵ are independently of each other selected from: -H, -CH₃, -C₂H₅, -C₃H₇, -C₄H₉, -C₅H₁₁, -C₆H₁₃, -OCH₃, -OC₂H₅, -OC₃H₇, -OC₄H₉, -SCH₃, -SC₂H₅, -SC₃H₇, -SC₄H₉, -NR¹⁸R¹⁹, -NR²⁰R²¹, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-C₃H₇, -CH₂-CH=CH-C₂H₅, -C₂H₄-CH=CH-CH₃, -C₃H₇-CH=CH₂, -CH=CH-CH=CH₂, -C=CH, -C≡C-CH₃, -CH₂-C≡CH, -C₂H₄-C≡CH, -CH₂-C≡C-CH₃, -C≡C-C₂H₅, -C≡C-C₃H₇, -CH₂-C≡C-C₂H₅, -C₂H₄-C≡C-CH₃, -C₃H₇-C≡CH,
R⁹, R¹⁶, R¹⁸, R¹⁹, R²⁰ and R²¹ are independently of each other selected from -H, -CH₃, -C₂H₅, -C₃H₇, -C₄H₉, -C₅H₁₁, -C₆H₁₃, 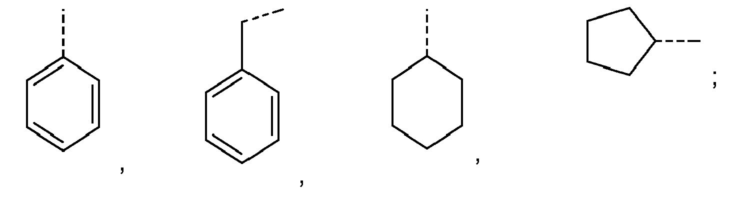 n and m are independently of each other selected from 1, 2, 3.
and enantiomers, stereoisomeric forms, hydrates, solvates, acid salt forms, tautomers, and racemates of the above mentioned compounds and pharmaceutically acceptable salts thereof for use in the therapeutic activation of autophagy.

2. Compound according to claim 1 for use in the treatment of a psychiatric disorder, infectious disease, a myopathy or a neurodegenerative disease.

3. Compound according to claim 2, wherein the psychiatric disorder is an affective disorder, schizophrenia or a sleep disorder.

4. Compound according to claim 3, wherein the affective disorder is selected from the group consisting of: depression, anxiety disorder, bipolar disorder, mania, substance induced mood disorder and seasonal affective disorder.

5. Compound according to claim 4, wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

6. Compound according to claim 2, wherein the infectious disease is a viral infection.

7. Compound according to claim 2, wherein the neurodegenerative disease is Alzheimer's disease, Parkinson's disease, Machado-Joseph disease or Huntington's disease.

8. Compound according to any one of claims 1 - 7 selected from the group consisting of:
*N*-(4-butyl-2-methylphenyl)acetamide and
4-butyl-2-methylaniline.

9. Pharmaceutical composition comprising at least one compound according to any one of claims 1 - 8 together with at least one pharmaceutically acceptable carrier, solvent or excipient.

10. A method for screening for a compound for the treatment of a psychiatric disease, infectious disease, a myopathy or a neurodegenerative disease, the method comprising
a) contacting a test compound with at least one polypeptide selected from the group consisting of Skp2, Beclin1 and Akt polypeptide,
b) detecting the binding of said test compound to the selected polypeptide, and
c) determining the activity or stability of the at least one polypeptide of step a) in the presence of said test compound.

11. The method according to claim 10, wherein instead of polypeptides nucleic acids encoding the polypeptides are used and the expression rate is determined instead of the activity.

12. A method of determining if a subject suffering from depression responds to an antidepressant, comprising:
measuring the protein level of at least one of FKBP51, Beclin1, pAkt^{S473}, pAkt^{S473}/Akt, pGsk3β^{S9}, pGsk3β^{S9}/Gsk3β and Atg12
in a blood sample from said subject comprising peripheral blood mononuclear cells from said subject, wherein at least one increased protein level of FKBP51, Beclin1, pGsk3β^{S9}, pGsk3β^{S9}/Gsk3β and Atg12 or a decreased level of pAkt^{S473} or pAkt^{S473}/Akt is indicative for a response to the antidepressant.

13. The method according to claim 12, wherein the protein level is increased in said sample compared to the protein level in a blood sample from the same subject taken before.

14. The method according to claim 12, for determining if a subject suffering from depression will respond to an antidepressant, comprising:
measuring the protein level of at least one of FKBP51, Beclin1, pDNMT1^{S154}/DNMT1, pAkt^{S473}, pAkt^{S473}/Akt, pGsk3β^{S9}, pGsk3β^{S9}/Gsk3β and Atg12 in peripheral blood mononuclear cells from a blood sample from said subject after incubation of the cells with the antidepressant,
wherein at least one increased protein level of FKBP51, Beclin1, pGsk3β^{S9}, pGsk3β^{S9}/Gsk3β and Atg12 or at least one decreased protein level of pAkt^{S473}, pAkt^{S473}/Akt and pDNMT1^{S154}/DNMT1 in the sample with the antidepressant is indicative for a response to the antidepressant.


SEQUENCE LISTING

<110>  MAX-PLANCK-GESELLSCHAFT zur FÖRDERUNG der WISSENSCHAFTEN
       e.V.

<120>  Small molecule inhibitors of the E3 Ligase Skp2 in depression and
        other diseases

<130>  MPP-P03685EP

<160>  4

<170>  PatentIn version 3.5

<210>  1
<211>  20
<212>  DNA
<213>  Artificial Sequence

<220>
<223>  antisense primer for Beclin1

<400>  1
gacgttgagc tgagtgtcca                                                   20


<210>  2
<211>  20
<212>  DNA
<213>  Artificial Sequence

<220>
<223>  sense primer for Beclin1

<400>  2
gtcactgggg acctttttga                                                   20


<210>  3
<211>  21
<212>  DNA
<213>  Artificial Sequence

<220>
<223>  antisense primer for beta-actin

<400>  3
caggtccaga cgcaggatgg c                                                 21


<210>  4
<211>  21
<212>  DNA
<213>  Artificial Sequence

<220>
<223>  sense primer for beta-actin

<400>  4
ctacaatgag ctgcgtgtgg c                                                 21